(19) Europäisches Patentamt
European Patent Office
Office européen des brevets

(11) Veröffentlichungsnummer: **0 254 185**
**B1**

(12) # EUROPÄISCHE PATENTSCHRIFT

(45) Veröffentlichungstag der Patentschrift: **19.09.90**

(51) Int. Cl.[5]: **C07C 271/24**, A61K 6/08, A61C 13/087

(21) Anmeldenummer: **87110146.5**

(22) Anmeldetag: **14.07.87**

(54) **Urethangruppen enthaltende (Meth)-acrylsäurederivate von Tricyclo [5.2.1.02.6]decanen.**

(30) Priorität: **25.07.86 DE 3625204**
**03.02.87 DE 3703120**

(43) Veröffentlichungstag der Anmeldung: **27.01.88 Patentblatt 88/4**

(45) Bekanntmachung des Hinweises auf die Patenterteilung: **19.09.90 Patentblatt 90/38**

(84) Benannte Vertragsstaaten: **AT BE CH DE ES FR GB IT LI NL SE**

(56) Entgegenhaltungen:
**DE-A- 2 931 926**
**DE-A- 3 245 563**
**US-A- 4 554 336**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Reiners, Jürgen, Dr., Carl-Rumpff Strasse 57, D-5090 Leverkusen 1(DE)**
Erfinder: **Podszun, Wolfgang, Dr., Wolfskaul 4, D-5000 Koeln 80(DE)**
Erfinder: **Winkel, Jens, Dr., Hahnenweg 6, D-5000 Koeln 80(DE)**

**EP 0 254 185 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Urethangruppen enthaltende (Meth)-acrylsäurederivate von Tricyclo-[5.2.1.0$^{2.6}$]-decanen , ihre Herstellung und ihre Verwendung als monomere Komponenten für Dentalwerkstoffe.

Die Verwendung von polyfunktionellen (Meth)-acrylsäurederivaten als Komponenten für Zahnfüllungsmaterialien ist bekannt. So werden in der EP-A 0 107 936 Acrylsäureester und Meth-acrylsäureester von Pentaerythrit beschrieben. Die dort beschriebenen Monomeren ergeben in Kombination mit anorganischen Füllstoffen Dentalwerkstoffe, die einen unerwünschten Polymerisationsschrumpf aufweisen, der zu einer Spaltbildung zwischen Zahn- und Füllungsmaterial führt.

In der US 4 554 336 werden Urethangruppen enthaltende (Meth)-acrylsäurederivate für Adhesive im Dentalbereich beschrieben, bei denen die Urethangruppen durch einen eine (Meth)-acrylatgruppe enthaltenden Rest substituiert sind. Diese Verbindungen zeigen als Komponenten in Dentalmassen unzureichende Eigenschaften, insbesondere eine für die Praxis zu geringe Festigkeit.

Es wurden neue Urethangruppen enthaltende (Meth)-acrylsäurederivate der Formel (I)

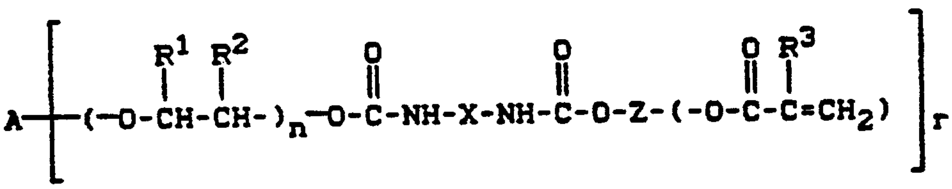 (I),

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X für die Gruppe

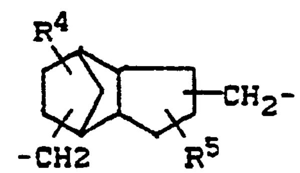

in der

$R^4$ und $R^5$ gleich oder verschieden sind und Wasserstoff, Halogen, Niederalkoxy, Niederalkyl oder Trifluormethyl bedeuten,

steht,

Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und

$R^3$ für jede von A ausgehende Kette unabhängig Wasserstoff oder Methyl bedeutet,

gefunden.

Dentalwerkstoffe, bei denen von den erfindungsgemäßen Urethangruppen enthaltenden (Meth)-acrylsäurederivaten von Tricyclo[5.2.1.0$^{2.6}$]decanen ausgegangen wird, zeigen überraschenderweise einen wesentlich geringeren Polymerisationsschrumpf und größere Festigkeit und sind daher für die Anwendung in der Praxis besonders geeignet.

Im Rahmen der vorliegenden Erfindung können die Substituenten folgende Bedeutung haben:

Ein aliphatischer Rest (A) kann ein geradkettiger oder verzweigter Kohlenwasserstoffrest mit 2 bis 20, bevorzugt 3 bis 12, Kohlenstoffatomen sein. Beispielsweise seien die folgenden aliphatischen Reste genannt:

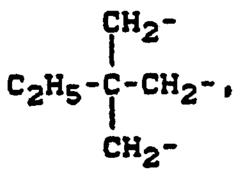, 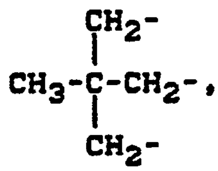, 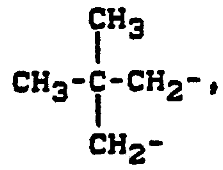, $-CH_2-CH_2-$,

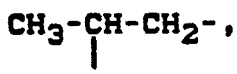, 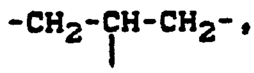, 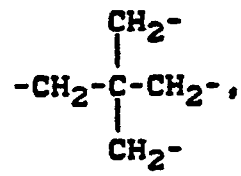,

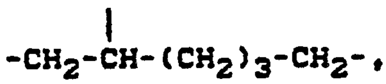, 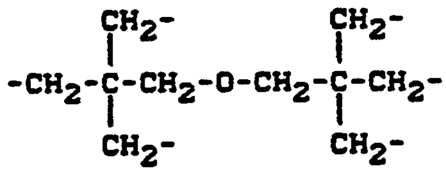

Ein aromatischer Rest (A) kann ein aromatischer Kohlenwasserstoffrest mit 6 bis 24, bevorzugt 6 bis 14, Kohlenstoffatomen sein. Beispielsweise seien die folgenden aromatischen Reste genannt:

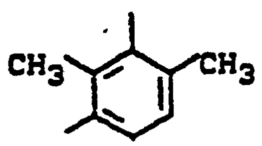, 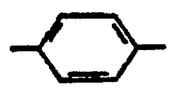, 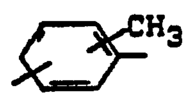,

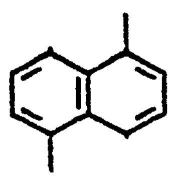, 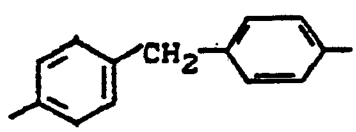

Ein araliphatischer Rest (A) kann ein Kohlenwasserstoffrest mit einem geradkettigen oder verzweigten aliphatischen und einem aromatischen Teil mit 7 bis 26 Kohlenstoffatomen bedeuten, wobei der aromatische Teil bevorzugt 6 bis 12 und der aliphatische Teil bevorzugt 1 bis 14 Kohlenstoffatome enthält. Beispielsweise seien die folgenden araliphatischen Reste genannt:

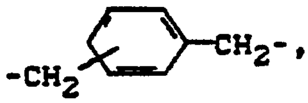

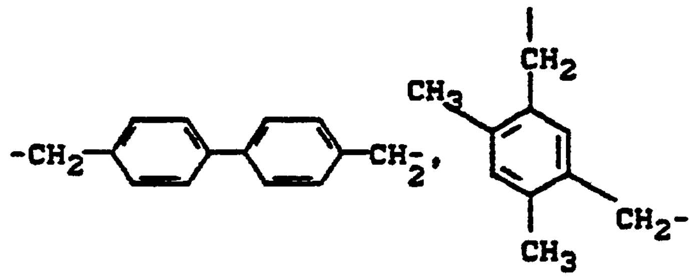

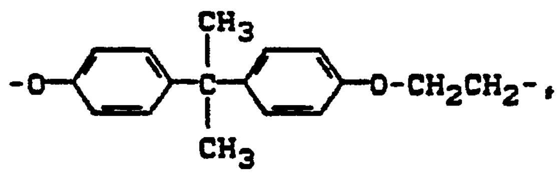

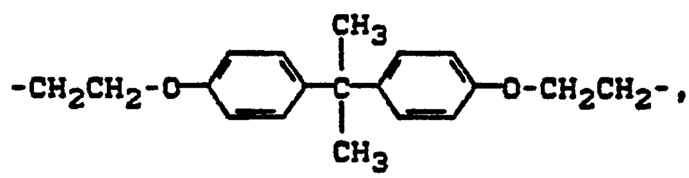

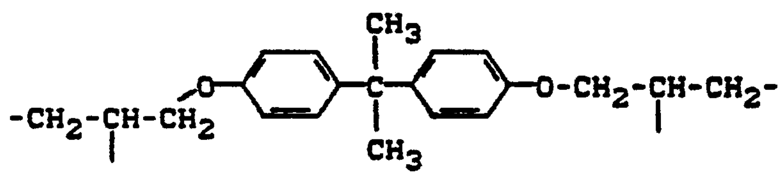

Ein cycloaliphatischer Rest (A) kann ein cyclisher Kohlenwasserstoffrest mit 6 bis 26 Kohlenstoffatomen, bevorzugt 6 bis 14 Kohlenstoffatomen, sein. Beispielsweise seien die folgenden cycloaliphatischen Reste genannt:

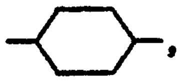, 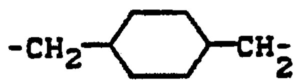, 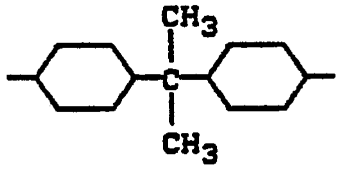,

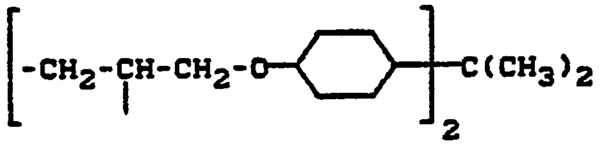, 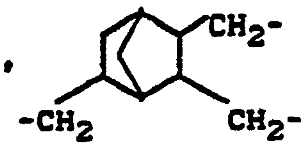,

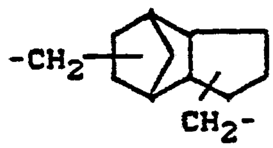, 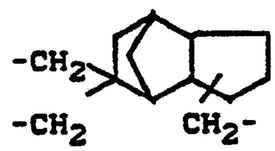,

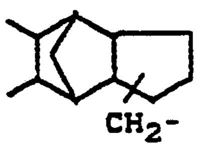, 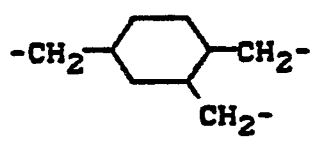, 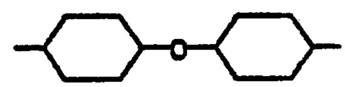

Die Reste A können, bevorzugt im aliphatischen oder cycloaliphatischen Teil, 1 oder 2, bevorzugt 1, Sauerstoffatome enthalten, so daß beispielsweise aliphatische bzw. cycloaliphatische Ether vorliegen.

Insbesondere bevorzugt seien die folgenden Reste A genannt: Ethylen, Propylen, 2,2-Bismethylenbutan-1-yl, 2,2-Bismethylen-propan-1-yl, 2,2-Bis-methylen-propan-1,3-diyl, 1,1'-Oxy-bis-[(2,2-methylen)-propan-1,3-diyl], Propan-1,2,3-triyl, 1,6-Hexamethylen, 1,4-Tetramethylen, 1,4-Phenylen, Xylylen, 1,4-Cyclohexylen, 1,4-Bismethylen-1,4-cyclohexan, 2,2-bis(1,4-phenylen)propan, 3(4), 8(9)-Bismethylen-tricyclo[5.2.1.$0^{2,6}$]decan und dessen Isomere, 4 oder 5,9-Bismethylen-3,8-dimethyltricyclo-[5.2.1.$0^{2,6}$]decan.

Insbesondere bevorzugt sind die Reste 2,2-Bismethylenbutan-1-yl, Propan-1,2,3-triyl, 2,2-Bismethylenpropan-1,3-diyl und 3(4),8(9)-Bismethylen-tricyclo[5.2.1.0.2,6]decan.

In der Gruppe $R^4$ beziehungsweise $R^5$ kann Niederalkyl einen geradkettigen oder verzweigten Kohlenwasserstoffrest mit 1 bis etwa 6 Kohlenstoffatomen bedeuten. Beispielsweise seien die folgenden Niederalkylreste genannt: Methyl, Ethyl, Propyl, Isopropyl, Butyl, tert. Butyl, Isobutyl, Pentyl, Isopentyl, Hexyl und Isohexyl. Bevorzugt werden der Methyl- und der Ethylrest.

Niederalkoxy kann ein über Sauerstoff gebundener geradkettiger oder verzweigter Kohlenwasserstoffrest mit 1 bis etwa 6 Kohlenstoffatomen bedeuten. Beispielsweise seien die folgenden Niederalkoxyreste genannt: Methoxy, Ethoxy, Propoxy, Isopropoxy, Butoxy, Isobutoxy, Pentoxy, Isopentoxy, Hexoxy und Isohexoxy. Bevorzugt sind der Methoxy- und der Ethoxyrest.

Halogen kann Fluor, Chlor, Brom oder Iod bedeuten. Bevorzugte Halogene sind Fluor und Chlor.

Als Gruppen X werden beispielsweise genannt:

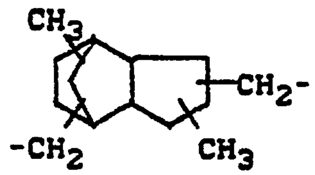

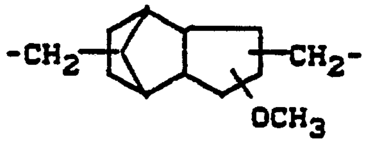

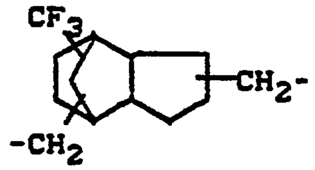

Bevorzugte Gruppe X ist der Rest

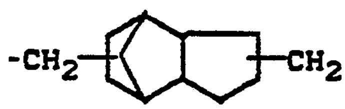

Ein zweiwertiger Kohlenwasserstoffrest (Z) kann ein geradkettiger oder verzweigter aliphatischer Kohlenwasserstoff mit 3 bis 15 Kohlenstoffatomen, bevorzugt 3 bis 10, Kohlenstoffatomen bedeuten. Der Rest Z kann gegebenenfalls 1 bis 3 Sauerstoffbrücken, bevorzugt 1 bis 2 Sauerstoffbrücken enthalten. Est ist auch möglich, daß der Rest Z durch 1 bis 4, bevorzugt 1 bis 2 (Meth)-acrylatreste substituiert ist. Beispielsweise seien die folgenden Reste genannt:

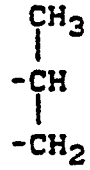 , 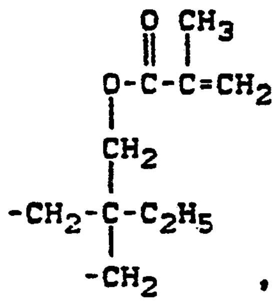 , 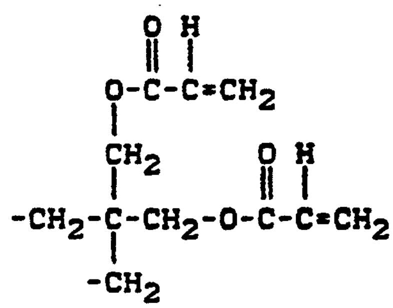 ,

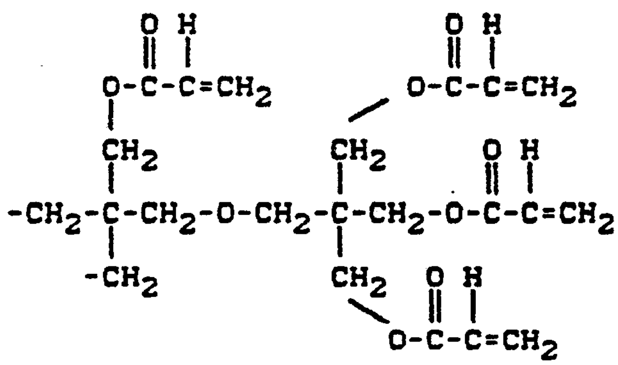 ,

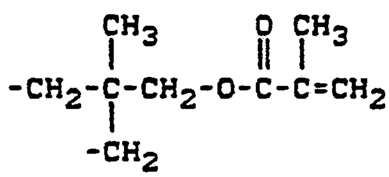 ,

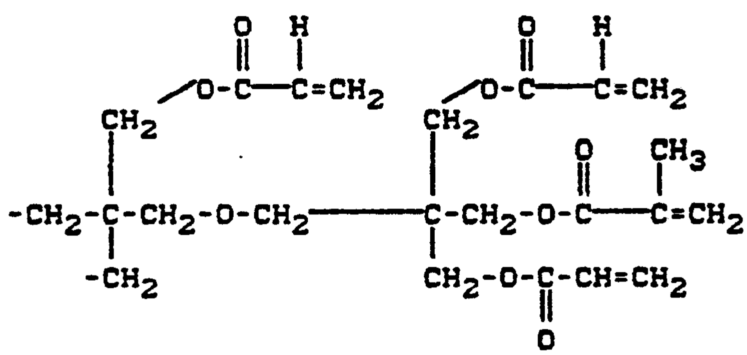 ,

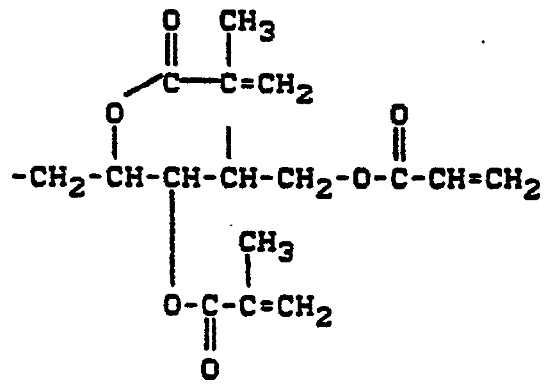

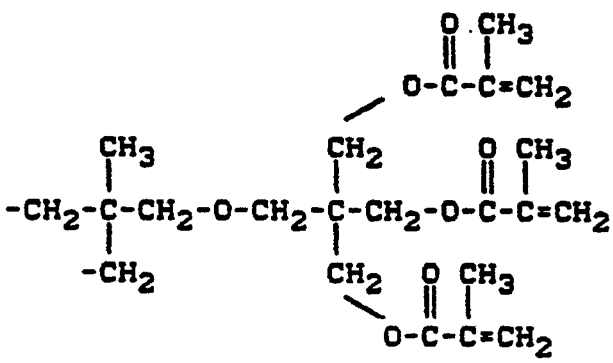

Bevorzugt werden Urethangruppen enthaltende (Meth)-acrylsäurederivate der Formel (I), bei denen
A ein geradkettiger oder verzweigter, gegebenenfalls 1 bis 3 Sauerstoffbrücken enthaltender, aliphatischer Rest mit 3 bis 12 Kohlenstoffatomen, ein aromatischer Rest mit 6 bis 14 Kohlenstoffatomen, ein araliphatischer Rest mit 7 bis 26 Kohlenstoffatomen oder ein cycloaliphatischer Rest mit 6 bis 14 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig einer Zahl von 0 bis 5 bedeutet,
X die Gruppe

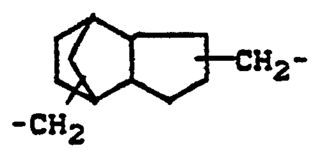

bedeutet,
Z einen zweiwertigen geradkettigen oder verzweigten aliphatischen Kohlenwasserstoffrest mit 3 bis 10 Kohlenstoffatomen, der gegebenenfalls 1 oder 2 Sauerstoffbrücken enthalten kann und gegebenenfalls durch 1 oder 2 (Meth)-acrylatreste substituiert sein kann, bedeutet, und
$R^3$ für jede von A ausgehende Kette unabhängig Wasserstoff oder Methyl bedeutet.

Insbesondere bevorzugt werden Urethangruppen enthaltende (Meth)-acrylsäurederivate der Formel (I), bei denen
A für den 2,2-Bismethylen-butan-1-yl-Rest, Propan-1,2,3-triyl-Rest, 2,2-Bismethylenpropan-1,3-diyl-Rest oder 3(4),8(9)-Bismethylen-tricyclo[5.2.1.$0^{2,6}$]decan-Rest steht,
r für die Anzahl der von A ausgehenden Ketten steht und die Zahl 3 oder 4 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X die Gruppe

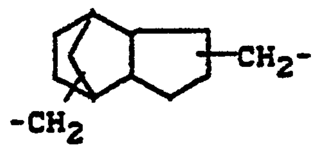

bedeutet,

Z einen zweiwertigen geradkettigen oder verzweigten aliphatischen Kohlenwasserstoffrest mit 3 bis 10 Kohlenstoffatomen, der gegebenenfalls 1 Sauerstoffbrücke enthalten und gegebenenfalls durch 1 (Meth)-acrylatrest substituiert sein kann, bedeutet, und

$R^3$ für jede von A ausgehende Kette unabhängig Wasserstoff oder Methyl bedeutet.

Beispielsweise seien die folgenden Urethangruppen enthaltenden (Meth)-acrylsäurederivate von Tricyclo $[5.2.1.0^{2.6}]$decanen genannt:

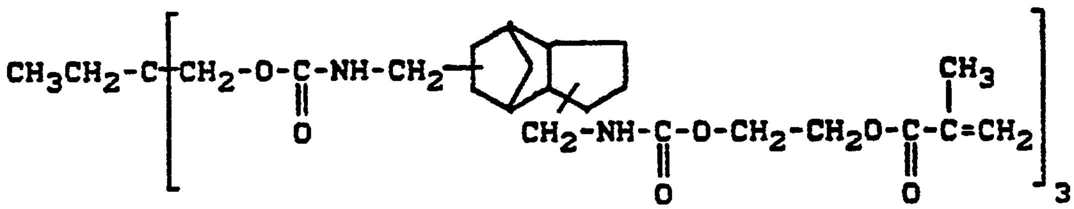

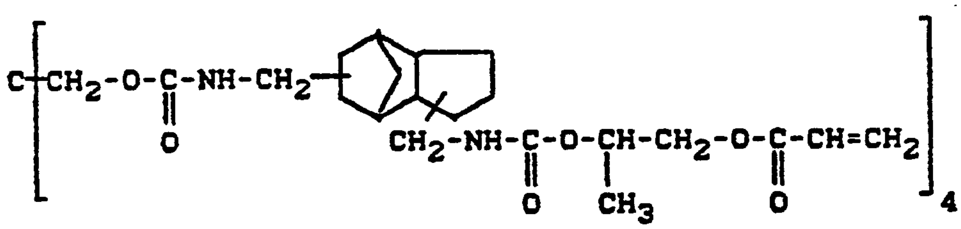

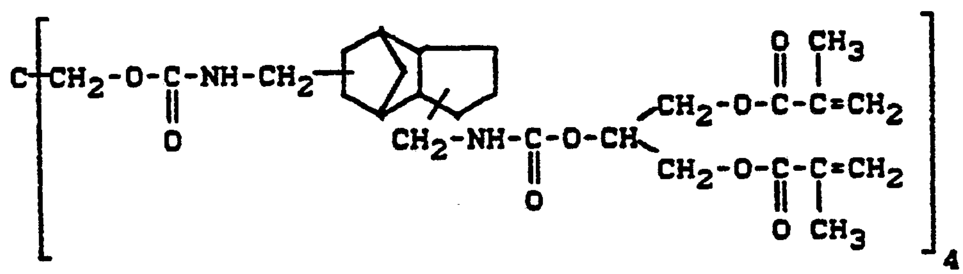

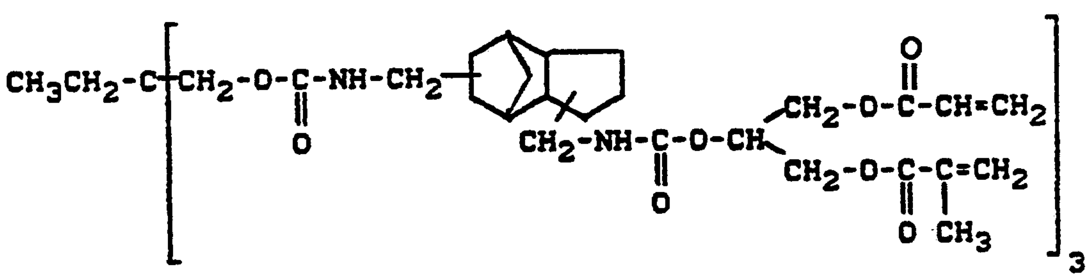

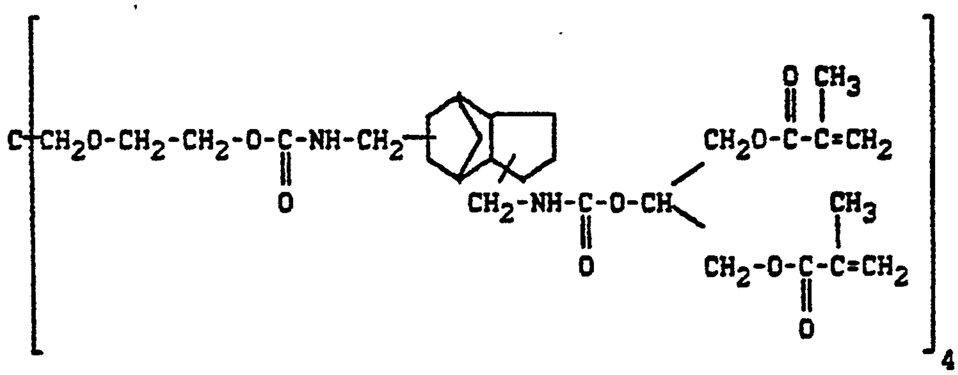

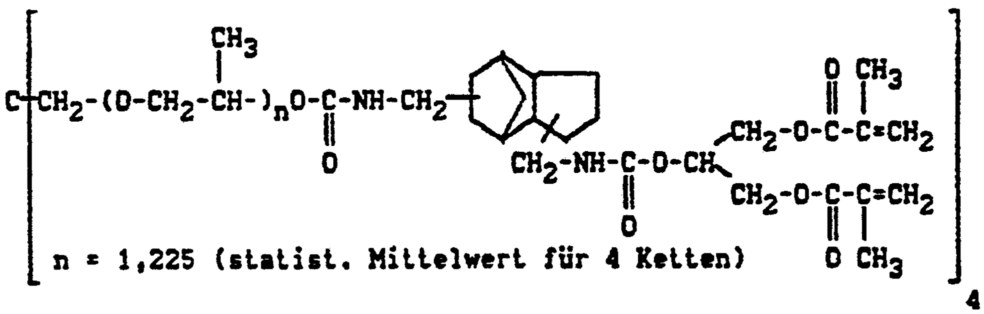

n = 1,225 (statist. Mittelwert für 4 Ketten)

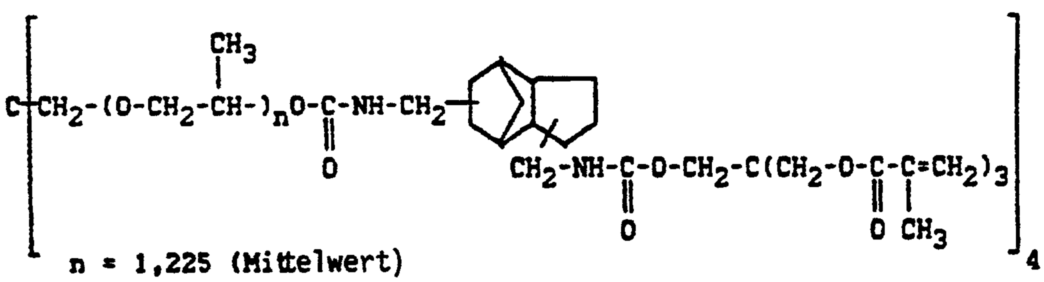

n = 1,225 (Mittelwert)

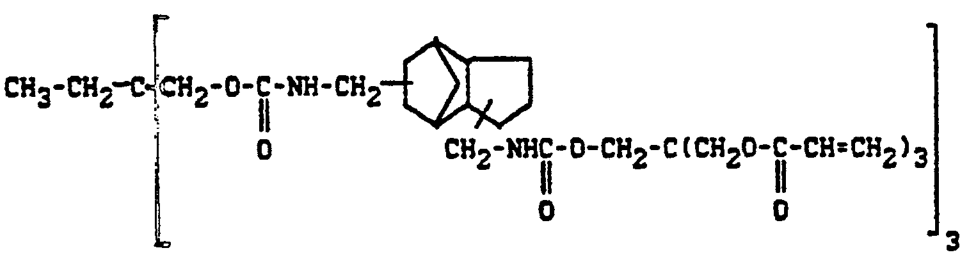

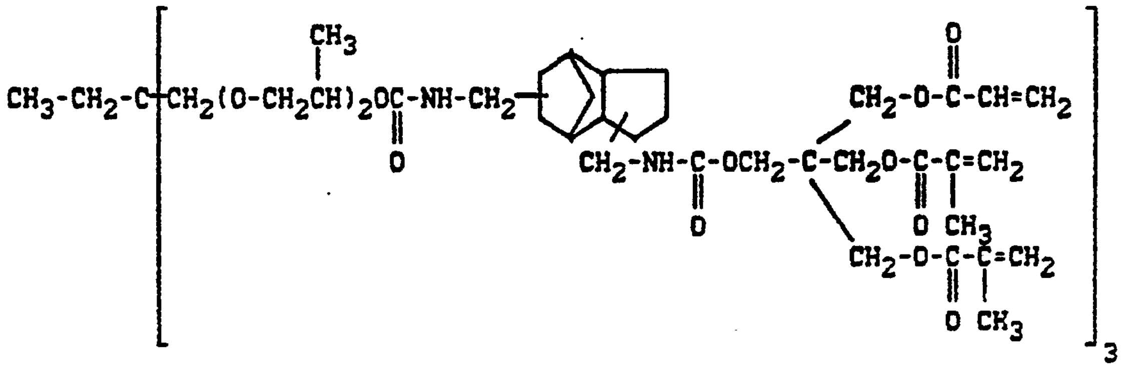

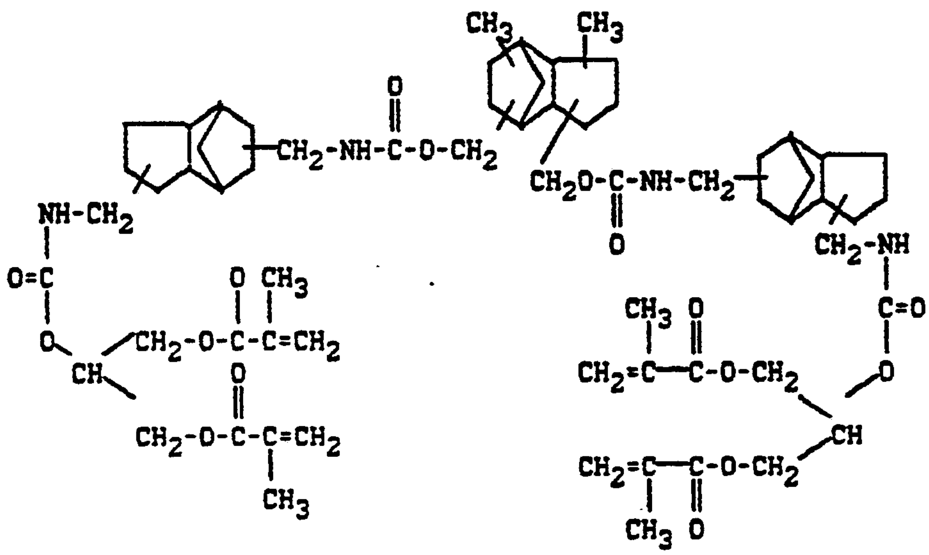

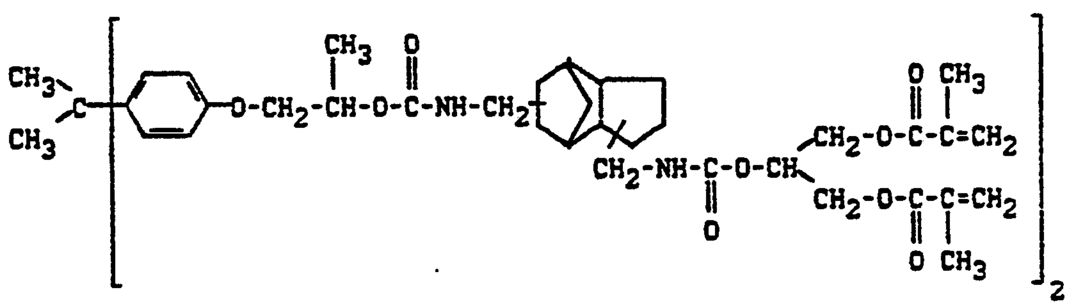

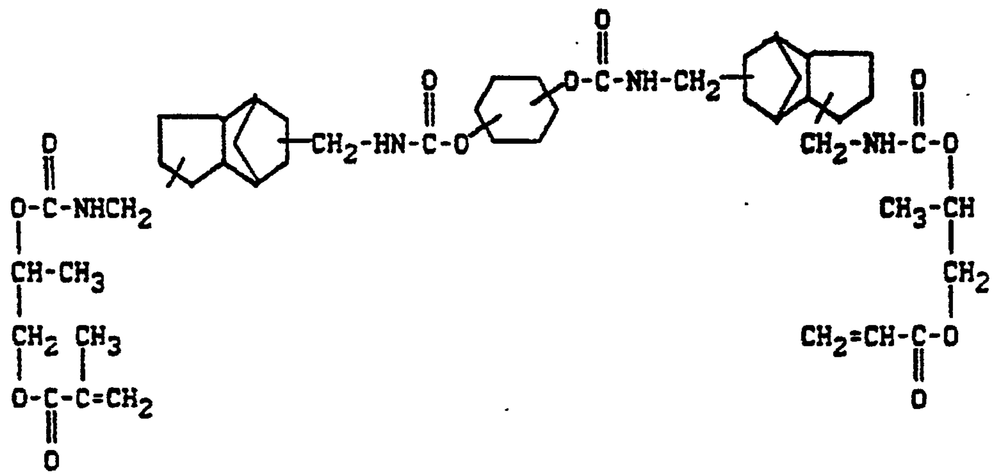

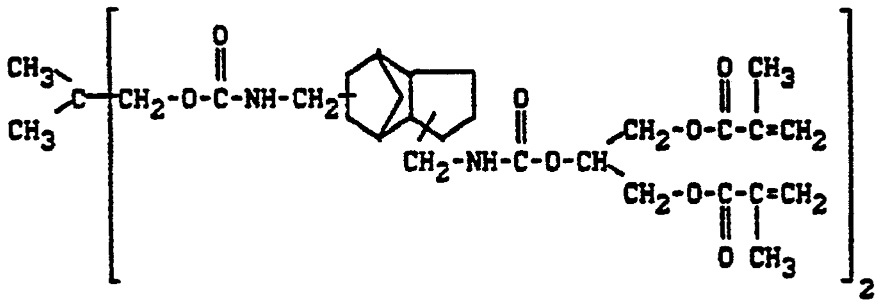

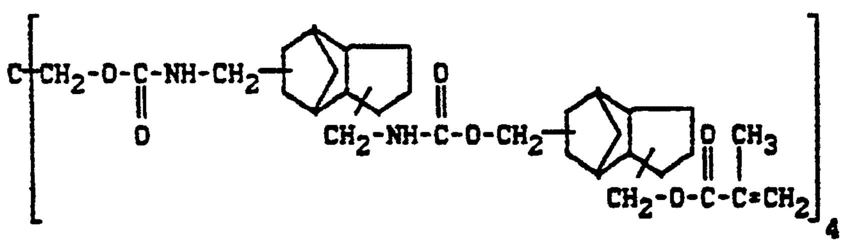

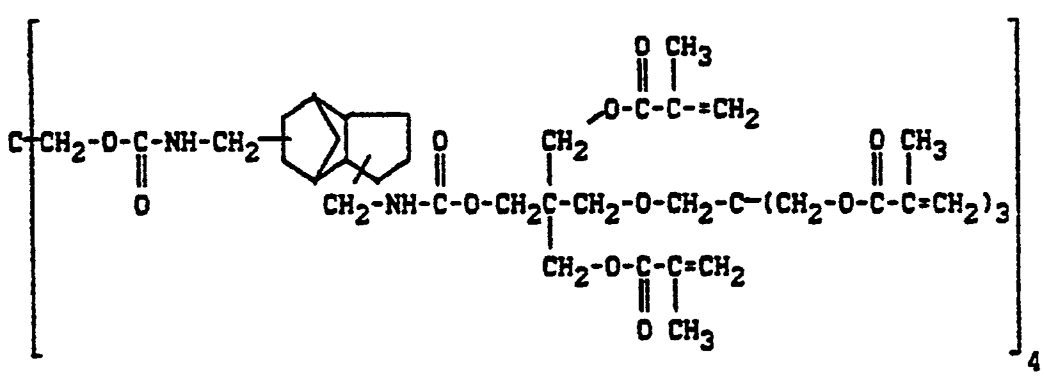

Es wurde auch ein Verfahren zur Herstellung der erfindungsgemäßen Urethangruppen enthaltenden (Meth)-acrylsäurederivate von Tricyclo[5.2.1.$0^{2.6}$]decanen der Formel (I)

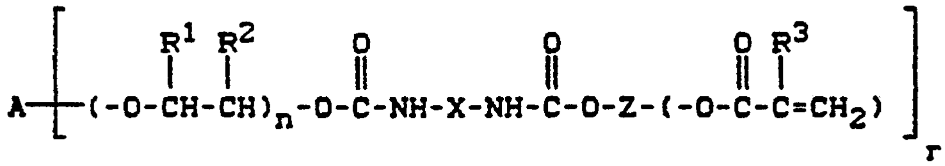 (I),

in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig einer Zahl von 0 bis 5 bedeutet,
X für die Gruppe

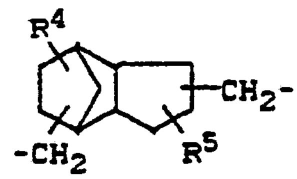 ,

in der
$R^4$ und $R^5$ gleich oder verschieden sind und Wasserstoff, Halogen, Niederalkoxy, Niederalkyl oder Trifluormethyl bedeuten,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 (Meth)-acrylatreste substituiert sein kann, bedeutet, und
$R^3$ für jede von A ausgehende Kette unabhängig Wasserstoff oder Methyl bedeutet,
dadurch gekennzeichnet, daß man einen Hydroxyalkyl-(Meth)-acrylsäureester der Formel (II)

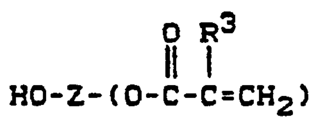 (II),

in der
Z und $R^3$ die obengenannte Bedeutung haben,
mit einen Diisocyanat der Formel (III)

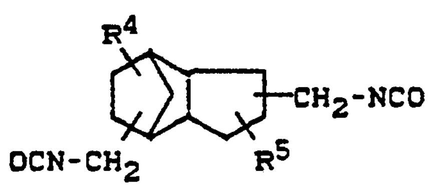 (III),

in der
$R^4$ und $R^5$ die obengenannte Bedeutung haben,
im Molverhältnis von etwa 1:1 bis 1:6 in einem inerten Lösungsmittel in Gegenwart eines Katalysators umsetzt und dann das entstandene Isocyanatourethan nach Entfernen von nicht umgesetztem Diisocyanat mit einem Polyol der Formel (IV)

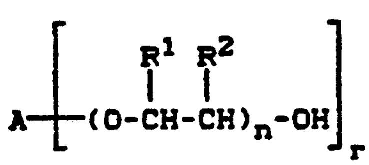 (IV),

in der
A, $R^1$, $R^2$, n und r die obengenannte Bedeutung haben,
im Movlerhältnis von OH-Gruppen zu NCO-Gruppen von etwa 1:1 umsetzt,
gefunden.

(Meth)-acrylsäureester der Formel II sind an sich bekannt und können beispielsweise durch partielle Veresterung der entsprechenden Polyole erhalten werden.

Diisocyanate von Tricyclodecanen der Formel III sind an sich bekannt und können beispielsweise durch Umsetzung der Diamine der Tricyclodecane mit Phosgen hergestellt werden.

Es ist zweckmäßig, das entstandene Isocyanatourethan zu reinigen, wenn das Diisocyanat III im Überschuß, bezogen auf den Hydroxyalkyl(meth)acrylsäureester II engesetzt wurde. Die Reinigung des Adduktes von II und III erfolgt bevorzugt durch Extraktion mit aliphatischen Lösungsmitteln mit Siedepunkten unter 120°C bei Normaldruck, z.B. mit Pentan, n-Hexan, Isopentan.

Polyole der Formel IV sind an sich bekannt (DE-A 2 931 925) bzw. handelsüblich und können beispielsweise durch Oxyalkylierung der bekannten Polyole der Formel $A(OH)_r$, z.B. 2,2-Bishydroxymethylbutan, 2,2-Bishydroxymethyl-propan-1,3-diol, 3(4),8(9)-Bishydroxymethyl-tricyclo[5.2.1.$0^{2,6}$]decan usw. hergestellt werden. Durch die Herstellung bedingt, können die Polyole IV auch als Produkt mit variablem Oxyalkylierungsgrad vorliegen.

Für das erfindungsgemäße Verfahren werden im allgemeinen inerte Lösungsmittel verwendet. Beispielsweise seien Aceton, Chloroform, Tetrahydrofuran, Dioxan, Methylenchlorid, Toluol, Acetonitril genannt. Besonders bevorzugt sind Chloroform, Toluol, Acetonitril und Aceton.

Im allgemeinen wird das erfindungsgemäße Verfahren unter Ausschluß von Wasser durchgeführt. Besonders bevorzugt wird eine maximale Menge an Wasser unter 0,1 Gew.-%, bezogen auf die Gesamtmenge der Reaktanden.

Katalysatoren für das erfindungsgemäße Verfahren sind im allgemeinen Metallsalze höherer Fettsäuren. Bevorzugte Katalysatoren können beispielsweise Dibutylzinnlaurat, Dibutylzinnmethoxid und Zinn-(II)-octoat sein. Als Katalysatoren können aber auch Verbindungen mit tertiären Aminogruppen, wie Triethylamin, Pyridin, 2-Methylpyridin, N,N-Dimethylpiperazin und N,N-Dimethyl-benzylamin verwendet werden. Es ist auch möglich, Titanverbindungen wie Tetrabutyl-titanat einzusetzen.

Im allgemeinen wird der Katalysator in einer Menge von 0,1 bis 2,5 Gew.-%, bevorzugt 0,1 bis 1,5 Gew.-%, bezogen auf die Gesamtmenge der Reaktanden eingesetzt werden.

In einer bevorzugten Ausführungsform kann das erfindungsgemäße Verfahren in Gegenwart eines Polymerisationsinhibitors durchgeführt werden. Polymerisationsinhibitoren sind an sich bekannt (Ullmanns Enzyklopädie der techn. Chemie, 4. Auflage, Verlag Chemie Weinheim, Band 8, Seiten 19-45). Beispielsweise seien 2,6-Ditert.-butyl-4-methylphenol, Hydrochinon, Hydrochinonmonomethylether genannt.

Es ist auch möglich, als Polymerisationsinhibitor Sauerstoff, z.B. Luftsauerstoff, zu verwenden, der in das Reaktionsgemisch eingeleitet wird.

Im allgemeinen wird der Polymerisationsinhibitor in einer Menge von 0,01 bis 1,0 Gew.-%, bevorzugt von 0,1 bis 0,2 Gew.-%, eingesetzt.

Die erste Stufe des erfindungsgemäßen Verfahrens wird im allgemeinen im Temperaturbereich von 0 bis 120°C, vorzugsweise von 30 bis 70°C, durchgeführt. Die zweite Stufe des erfindungsgemäßen Verfahrens wird im allgemeinen im Temperaturbereich von 0 bis 120°C, vorzugsweise von 30 bis 70°C, durchgeführt.

Das erfindungsgemäße Verfahren wird im allgemeinen bei Normaldruck durchgeführt. Es ist aber auch möglich, das erfindungsgemäße Verfahren bei Unter- oder Überdruck (beispielsweise im Druckbereich von 0,1 bis 10 bar) durchzuführen.

Das erfindungsgemäße Verfahren kann beispielsweise wie folgt durchgeführt werden:

Der (Meth)-acrylsäureester der Formel (II) und gegebenenfalls der Polymerisationsinhibitor werden im inerten Lösungsmittel gelöst und unter Rühren zu dem gegebenenfalls gelösten Diisocyanat (III) zugetropft. Der Katalysator wird dabei einem der beiden Reaktanden zugesetzt. Die Reaktanden werden im Molverhältnis von etwa 1:1 bis 1:6 zur Umsetzung gebracht und bis zum vollständigen Umsatz der OH-Gruppen bzw. zum entsprechenden Umsatz der Isocyanatgruppen geführt. Die Umsetzung der Isocyanatgrupen kann in bekannter Weise durch IR-Spektroskopie und/oder durch Titration kontrolliert werden.

Ein Überschuß Diisocyanat kann anschließend mit n-Hexan, n-Pentan oder anderen aliphatischen Lösungsmitteln mit einem Siedepunkt unter 120°C (bei Normaldruck) extrahiert werden.

In der zweiten Stufe des erfindungsgemäßen Verfahrens wird das in der ersten Stufe erhaltene Isocyanatourethan gegebenenfalls nach Extraktion eventuell vorhandenen überschüssigen Diisocyanats mit einem Polyol der Formel IV umgesetzt, so daß die Anzahl der Hydroxyläquivalente des Polyols etwa der Anzahl der noch vorhandenen NCO-Äquivalente entspricht.

Bevorzugt werden

$$\frac{0,9}{r} \text{ bis } \frac{1,1}{r}$$

Mol der Polyols IV, bezogen auf 1 Mol Hydroxyalkyl(meth)acrylat II, eingesetzt, r hat hierbei die oben angegebene Bedeutung einer Zahl von 2 bis 6.

Die Reaktion wird im allgemeinen bis zum vollständigen Umsatz geführt, so daß weder freies Isocyanat

noch Polyol in der Umsetzung verbleiben. Nach beendeter Umsetzung wird das Reaktionsprodukt durch Entfernen des Lösungsmittels isoliert. Eine vorherige Filtration oder Reinigung mit Hilfe von Adsorbentien, bzw. Aktivkohle, Bleicherde, Kieselgel oder Aluminiumoxid ist möglich.

Nach dem erfindungsgemäßen Verfahren entsteht in der Regel ein Gemisch von Urethangruppen enthaltenden (Meth)-acrylsäurederivaten, die an Adsorbentien aufgetrennt werden können.

Es ist auch möglich, die erste und die zweite Stufe des obengenannten Verfahrens in ihrer Reinhenfolge zu vertauschen. In diesem Fall werden in der ersten Stufe Diisocyanat III und Polyol IV im Molverhältnis NCO:OH = 2 bis 10, vorzugsweise im Movlerhältnis NCO:OH = 2,0 bis 4, bis zur Umsetzung aller Hydroxylgruppen in Urethangruppen zur Reaktion gebracht.

Anschließend wird ein eventuell vorhandener Überschuß an Diisocyanat (sofern dieses im Überschuß eingesetzt wurde) in der oben beschriebenen Weise mit den genannten Lösungsmitteln extrahiert. Die verbleibenden NCO-Gruppen werden dann in der zweiten Stufe mit einem Hydroxyalkyl(meth)acrylat II zum erfindungsgemäßen (Meth)acrylsäureester umgesetzt, wobei eine stöchiometrische Äquivalenz von NCO- und OH-Gruppen besteht.

Für den erfindungsgemäßen Einsatz der neuen Urethan(meth)acrylate auf dem Dentalgebiet ist eine Auftrennung der erhaltenen Reaktionsgemische nicht erforderlich. Die Gemische selbst können in vorteilhafter Weise als Komponente von Dentalwerkstoffen, beispielsweise Zahnfüllungsmaterialien, verwendet werden.

Die erfindungsgemäßen Urethan(meth)acrylate von Tricyclo[5.2.1.$0^{2,6}$]decanen können als Monomere für Dentalwerkstoffen verwendet werden. Als Dentalwerkstoffe seien beispielsweise Füllungsmaterialien für Zähne, Beschichtungsmittel für Zähne und Komponenten für die Herstellung von Zahnersatz, bevorzugt Kunststoffzähne, genannt. Je nach Anwendungsgebiet können Dentalwerkstoffe weitere Additive enthalten.

Für die Anwendung als Monomere für polymerisierbare Zahnfüllmassen oder Beschichtungsmittel im Dentalbereich können die erfindungsgemäßen (Meth)-acrylsäurederivate mit an sich bekannten Monomeren gemischt werden, um beispielsweise die Viskosität dem Verwendungszweck anzupassen. Hierbei werden Viskositäten im Bereich von 60 bis 10.000 mPas bevorzugt. Dieses ist dadurch erreichbar, daß man den erfindungsgemäßen Monomeren gegebenenfalls ein Comonomer niedriger Viskosität als Reaktivverdünner bzw. Lösungsmittel mischt. Die erfindungsgemäßen Verbindungen werden in der Mischung mit Comonomeren mit einem Anteil von ca. 30 bis ca. 90 Gew.-%, bevorzugt von 40 bis 80 Gew.-%, eingesetzt. Es ist ebenfalls bevorzugt, Mischungen verschiedener erfindungsgemäßer (Meth)-acrylsäureester einzusetzen.

Es ist auch möglich, Monomermischungen einzusetzen, die mehrere Comonomere enthalten, um die gewünschte Viskosität zu erreichen.

Beispielsweise seien die folgenden Comonomeren genannt: Glycerindi(meth)acrylat, Triethylenglykoldi(meth)acrylat, Tetraethylenglykoldi(meth)acrylat, 1,12-Dodecandioldi(meth)acrylat, 1,6-Hexandioldi(meth)acrylat, Diethylenglykol dimethacrylat, 2,2-Bis-[p-(2'-hydroxy-3'-methacryloyloxy propoxy)-phenyl]-propan, 2,2-Bis-[p-(2'-methacryloyloxyethoxy)-phenyl]-propan, Tri-methylol-propan-tri-(meth)-acrylat, Bis-(meth)-acryloyloxyethoxymethyl-tricyclo-[5,2,1,$0^{2,6}$]-decan (DE-A 29 31 925 und DE-A 29 31 926).

Insbesondere bevorzugt werden Comonomere, die bei 13 mbar einen Siedepunkt über 100°C besitzen.

Die erfindungsgemäßen polyfunktionellen (Meth)-acrylsäureester können gegebenenfalls in Mischung mit den genannten Comonomeren mit an sich bekannten Methoden zu vernetzten Polymerisaten aushärten (Am. Chem. Soc., Symp. Ser.212, 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentrationen des Peroxids bzw. des Amins werden vorteilhaft so gewählt, daß sie 0,1 bis 5 Gew.-%, bevorzugt 0,5 bis 3 Gew.-%, bezogen auf die Monomermischung betragen. Die peroxid- bzw. aminhaltigen Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die photoinitierte Polymerisation eignen sich beispielsweise Benzoin, Benzoindimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE-A 3 135 115 beschrieben.

Neben den oben beschriebenen Initiatoren können den erfindungsgemäßen (Meth)-acrylsäure-Derivaten an sich für diesen Einsatzzweck bekannte Lichtschutzmittel und Stabilisatoren zugesetzt werden.

Lichtschutzmittel sind beispielsweise in (Gächter, Müller, Taschenbuch der Kunststoff-Additive, 2. Ausgabe, Carl Hanser Verlag) beschrieben. Beispielsweise seien die folgenden Lichtschutzmittel genannt: Cyasorb UV9®, Tinuvin P®, Tinuvin 770®, Tinuvin 622®, Tinuvin 765®.

Stabilisatoren sind beispielsweise in (Ullmanns Encyclopädie der technischen Chemie, 4. Auflage, Bd. 8) beschrieben. Beispielsweise seien die folgenden Stabilisatoren genannt: 2,6-Di-tert.-butylphenol, 2,6-Di-tert.-butyl-4-methylphenol, 2,6-Di-octadecyl-4-methyl-phenol, 1,1'-Methylen-bis(naphthol-2) u.a.

Die Lichtschutzmittel und die Stabilisatoren können jeweils in einer Menge von 0,01 bis 0,5 Gew.-Teilen, bezogen auf 100 Gew.-Teile der Monomermischung, eingesetzt werden.

Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPas besitzen, besonders vorteilhaft.

Vorzugsweise mischt man den erfindungsgemäßen (Meth)-acrylsäurederivaten anorganische Füllstoffe zu. Beispielsweise seien Bergkristall, Kristoballit, Quarzglas, hochdisperse Kieselsäure, Aluminiumoxid und Glaskeramiken, beispielsweise Lanthan und Zirkon enthaltende Glaskeramiken (DE-A 23 47 591) genannt. Die anorganischen Füllstoffe werden zur Verbesserung des Verbunds zur Polymermatrix des Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297-308 (1983)). Bevozugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen Teilchendurchmesser und/oder einen unterschiedlichen Silangehalt besitzen.

Der Füllstoffanteil in der Zahnfüllmasse beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen vermischt.

Der Anteil der erfindungsgemäßen Urethan(meth)acrylate in den Zahnfüllmassen beträgt im allgemeinen 5 bis 70 Gew.-% bezogen auf die Füllmasse.

Die erfindungsgemäßen Urethan-(Meth)-Acrylsäuren-Derivate von Tricyclo-[5.2.1.0$^{2,6}$]decanen können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perlpolymerisate zugesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunststoffzähne werden duch radikalische Polymerisation der Dentalmassen unter Formgebung hergestellt.

Die Verarbeitung ist sowohl durch Injektionsverfahren als auch durch Prägeverfahren möglich und erfolgt im allgemeinen nach den üblichen Herstellungsmethoden für Zähne auf Basis von Poly(methylmethacrylat), z.B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azobisisobuttersäurenitril. Gut geeignet sind auch Mischungen von Polymerisationsinitiatoren mit unterschiedlichen Zerfallstemperaturen.

Die aus den erfindungsgemäßen (Meth)Acrylsäureestern hergestellten Dentalwerkstoffe zeichnen sich durch hohe Widerstandsfähigkeit gegenüber mechanischer Beanspruchung und eine hohe Abrasionsbeständigkeit aus.

Herstellungsbeispiele

Beispiel 1

Reaktion des (1:1)-Addukts aus Glycerindimethacrylat und Bis-(isocyanatomethyl)-tricyclo[5.2.1.0$^{2,6}$]-decan mit Pentaerythrit.

22,8 g handelsübliches Glycerindimethacrylat, 0,1 g Dibutylzinndilaurat und 20 mg 2,6-Di-tert.-butyl-4-methylphenol (Jonol) werden in 30 ml getrocknetem Methylenchlorid gelöst und zu 24,6 g Bis-isocyanatomethyl-tricyclo[5.2.1.0$^{2,6}$]decan bei 40 bis 45°C zugetropft. Es wird bei dieser Temperatur gerührt, bis die Hälfte der NCO-Gruppen umgesetzt ist. Die Bestimmung der NCO-Gruppen erfolgt in bekannter Weise durch Umsetzung mit Dibutylamin und Rücktitration des überschüssigen Dibutylamins mit Salzsäure. Bei dem gewünschten Umsatz werden weitere 30 ml Methylenchlorid und 3,4 g Pentaerythrit zugegeben. Die Mischung wird (etwa 48 h) bei 40 bis 45°C gerührt, bis im IR-Spektrum kein Isocyanat mehr feststellbar ist. Das Reaktionsprodukt wird über Aktivkohle oder Kieselgel filtriert und vom Lösungsmittel befreit. Es wird ein weißer Feststoff erhalten.

Schmelzpunkt 70 bis 75°C.

80 MHZ-1-H-NMR-Spektrum ($CDCl_3$) [ppm]:

| | | |
|---|---|---|
| 0,8 - 2,5 | Protonen des Tricyclodecansystems, | 56 H |
| 1,94 | 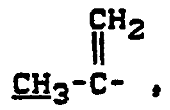 , | 24 H |
| 3,0 | 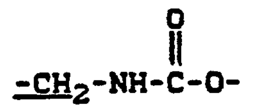 , | 16 H |
| 4,0 - 4,4 | 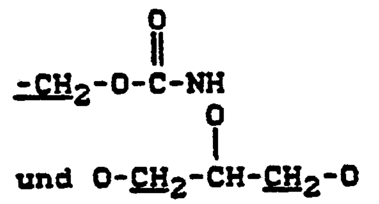 , | 24 H |
| 4,8 - 5,0 | NH, | 8 H |
| 5,1 - 5,4 | 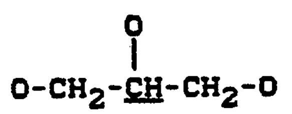 , | 4 H |
| 5,5 - 5,65 und 6,05 - 6,25 | 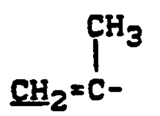 , | 16 H |

Beispiel 2

Reaktion des (1:1)-Addukts aus Glycerindimethacrylat und Bis-isocyanatomethyl-tricyclo[5.2.1.$0^{2.6}$]-decan mit Trimethylolpropan.

22,8 g Glycerindimethacrylat, 20 mg Ionol und 0,1 g Dibutylzinndilaurat werden in 30 ml getrocknetem Chlorform gelöst und zu 24,6 g Bis-isocyanatomethyl-tricyclo[5.2.1.$0^{2.6}$]decan bei 40 bis 50°C zugetropft. Es wird bei 40 bis 50°C gerührt, bis die Hälfte der NCO-Gruppen reagiert hat (ca. 3 Stunden). Dann werden 4,46 g Trimethylolpropan in 30 ml Chloroform zugegeben.

Man hält bis zum vollständigen Umsatz der NCO-Gruppen (IR-spektroskopische Kontrolle) eine Reaktionstemperatur von 40 bis 50°C aufrecht. Nach beendeter Reaktion wird gegebenenfalls über Kieselgel filtriert und das Lösungsmittel am Rotationsverdampfer abgetragen. Das Produkt ist ein farbloser Feststoff.

$^1$H-NMR-Spektrum in $CDCl_3$/TMS [ppm]:

| | | |
|---|---|---|
| 0,7 - 2,6 | Protonen des Tricyclodecan-Gerüsts<br>und $CH_2$-$CH_3$-C- , | 47 H |
| 1,94 | $CH_2$<br>‖<br>$\underline{CH}_3$-C- , | 18 H |
| 3,0 | O<br>‖<br>-$CH_2$-NH-C-O- , | 12 H |
| 3,9 - 4,1<br>und<br>4,2 - 4,45 | O<br>‖<br>-$CH_2$-O-C- , | 18 H |
| 4,8 - 5,1 | NH, | 6 H |
| 5,15 - 5,45 | $CH_2$O-<br>O-$\underline{CH}$ ,<br>$CH_2$O- | 3 H |
| 5,5 - 5,65<br>und<br>6,05 - 6,2 | $CH_3$<br>\|<br>$\underline{CH}_2$=C- , | 12 H |

Beispiel 3

Reaktion des (1:1)-Addukts aus Glycerindimethacrylat und Bis-isocyanatomethyl-tricyclo-[5.2.1.$0^{2.6}$]decan mit Bis-hydroxymethyl-tricyclo[5.2.1.$0^{2.6}$]decan (TCD-DM).

22,8 g Glycerindimethacrylat, 23 mg Ionol und 0,1 g Dibutylzinndilaurat werden in 30 ml getrocknetem Methylenchlorid gelöst und bei 40 bis 45°C in 24,6 g Bis-isocyanatomethyl-tricyclo[5.2.1.$0^{2.6}$]decan eingetropft. Nach Umsetzung der Hälfte der Isocyanatgruppen werden 9,8 g TCD-DM in 30 ml Methylenchlorid zugegeben. Nach etwa 24 Stunden bei 40 bis 45°C sind alle NCO-Gruppen umgessetzt. Das Lösungsmittel wird am Rotationsverdampfer entfernt. Das Produkt wird als farbloser Feststoff isoliert.
Schmelzpunkt: 65 bis 70°C
Molmasse (osmometrisch: 1.104 (ber. 1.144).

Beispiel 4

Reaktion des (1:1)-Addukts aus 2-Hydroxyethylmethacrylat (HEMA) und Bis-isocyanatomethyl-tricyclo-[5.2.1.$0^{2.6}$]decan mit Trimethylolpropan.

26 g entwässertes HEMA wird in 53 g Chloroform gelöst und nach Zugabe von 34 mg Ionol bei 45°C in 49,2 g Bis-isocyanatomethyl-tricyclo[5.2.1.$0^{2.6}$]decan eingetropft. Nach etwa 24 Stunden ist die Hälfte der NCO-Gruppen umgesetzt. Dann werden 8,9 g Trimethylolpropan und 0,1 g Zinnoctoat zugegeben und bei 45°C weitere 24 Stunden gerührt. Das Reaktionsgemisch wird über Kieselgel filtriert und wird nach Zugabe von 80 g Triethylenglykoldimethacrylat (TEGDMA) vom Lösungsmittel bis zur Gewichtskonstanz befreit. Das Produkt besitzt bei 25°C eine Viskosität von etwa 1.000 mPa.s.

Beispiel 5

Reaktion des (1:1)-Addukts aus Glycerindimethacrylat und Bis-isocyanatomethyl-tricyclo-[5.2.1.$0^{2.6}$]decan mit propoxyliertem Pentaerythrit.

34,2 g Glycerindimethacrylat, 34 mg Ionol und 0,1 g Zinnoctoat werden in 51 g Chloroform gelöst und bei 50°C in 36,9 g Bis-isocyanatomethyl-tricyclo[5.2.1.$0^{2.6}$]decan eingetropft. Nach Umsatz der Hälfte

der NCO-Gruppen werden 15,8 g des Addukts von 1 Mol Pentaerythrit und 4,9 Mol Propylenoxid (OH-Zahl = 534 mg KOH/g) zugegeben. Nach etwa 18 Stunden bei 50°C ist das Isocyanat vollständig umgesetzt. Man gibt 50 g TEGDMA zu und entfernt das Lösungsmittel im Vakuum bis zur Gewichtskonstanz.

Beispiel 6

44,7 g Pentaerythrittriacrylat und 35 mg Ionol werden in 91 g Chloroform gelöst und bei 50°C in 36,9 g Bis-isocyanatomethyl-tricyclo[5.2.1.$0^{2.6}$]decan und 50 mg Zinn(II)octoat eingetropft. Wenn die Hälfte der NCO-Gruppen umgesetzt ist, werden 6,7 g Trimethylolpropan in 50 ml Chloroform zugegeben und bis zum vollständigen Umsatz der NCO-Gruppen bei 50°C gerührt. Dann wird mit A-Kohle verrührt. Das Filtrat wird nach Zusatz von 97,6 g TEGDMA im Vakuum bis zur Gewichtskonstanz eingeengt.

Anwendungsbeispiele

Beispiel 7

Herstellung von Sealer-Lösungen

a) Lichthärtender Zahnlack (Sealer)

In einer Mischung aus 45 Gew.-Teilen Triethylenglycoldimethacrylat und 55 Gew.-Teilen Monomer aus Beispiel 1 werden 0,5 % N,N-Diallyl-p-dimethylaminobenzolsulfonsäureamid, 0,2 % Campherchinon und 0,125 % Benzildimethylketal gelöst.

Beim Bestrahlen mit einer handelsüblichen Dentallampe (Translux, Fa. Kulzer) härtet die Flüssigkeit zu einem festen Kunststoff aus. Diese gehärtete Sealerlösung besitzt nach DIN 13 922 eine Biegefestigkeit von 95 $N/mm^2$ und einen E-Modul von 2.100 $N/mm^2$.

Die entsprechend hergestellte Sealerlösung aus 59,9 Gew.-Teilen Monomer aus Beispiel 3) und 40,1 Gew.-Teilen Triethylenglycoldimethacrylat besitzt eine Biegefestigkeit von 105 $N/mm^2$ und einen E-Modul von 2.460 $N/mm^2$.

b) Redoxhärtendes System

Katalysatorlösung

In einer Mischung aus 45 Gew.-Teilen Triethylenglycoldimethacrylat und 55 Gew.-Teilen Monomer aus Beispiel 1 werden 2 % Benzoylperoxid gelöst.

Aktivatorlösung

In einer Mischung aus 45 Gew.-Teilen Triethylenglycoldimethacrylat und 55 Gew.-Teilen Monomer aus Beispiel 1 werden 2,15 % N-Methyl-N-β-(methylcarbamoyloxy)-propyl-3,5-dimethylanilin gelöst.

Eine Mischung aus gleichen Teilen Katalysatorlösung und Aktivatorlösung härtet in 1 Minute aus.

Beispiel 8

Masse zum Füllen von Zahnhohlräumen

a) Redoxhärtendes System

Peroxidpaste

In einer Mischung aus 55 Gew.-Teilen Monomer aus Beispiel 1 und 45 Gew.-Teilen Triethylenglycoldimethacrylat werden 2 % Benzoylperoxid gelöst. 10 g silanisierte Glaskeramik werden mit 4 g dieser Lösung zu einer Paste verarbeitet.

Aminpaste

In einer Mischung von 55 Gew.-Teilen Monomer aus Beispiel 1 und 45 Gew.-Teilen Triethylenglycoldimethacrylat werden 1,3 % N-Methyl-N-β-(methylcarbamoyloxy)-propyl-3,5-dimethylanilin gelöst.

4 g dieser Lösung werden mit 10 g silanisierter Glaskeramik zu einer Paste verarbeitet. Werden gleiche Teile Aminpaste und Peroxidpaste miteinander gemischt, so härtet die Mischung in 2 Minuten aus. Die Pasten können mit Pigmenten eingefärbt werden und eignen sich zum Füllen von Zahnhohlräumen.

b) Lichthärtendes System

In einer Mischung von 55 Gew.-Teilen Monomer aus Beispiel 1 und 45 Gew.-Teilen Triethylenglycoldimethacrylat werden 0,5 % N,N-Diallyl-p-dimethylaminobenzolsulfonsäure-amid, 0,2 % Campherchinon und 0,125 % Benzildimethylketal gelöst. 10 g silanisierte Glaskeramik werden mit 4 g dieser Lösung zu einer Paste verarbeitet. Bestrahlt man diese Masse mit einer handelsüblichen Dentallampe (Translux, Fa. Kulzer), so ist nach 40 sec. eine Schicht von 7 mm durchgehärtet.

Die lichthärtende Paste wird gemäß DIN 13 922 zu einem Dentalkunststoff ausgehärtet, der eine Biegefestigkeit von 135 $N/mm^2$ und einen E-Modul von 12.458 $N/mm^2$ ergibt.

Die analog hergestellte Paste aus 60 Gew.-Teilen Monomer aus Beispiel 2 und 40 Gew.-Teilen Triethylenglycoldimethacrylat ergibt eine Biegefestigkeit von 124 $N/mm^2$ und einen E-Modul von 11.107 $N/mm^2$.

Beispiel 9

Wallace-Härteprüfung von gehärten Sealerlösungen

Die Urethan-(Meth)acrylate von Tricyclodecanen aus den Herstellungsbeispielen werden mit Triethylenglycoldimethacrylat auf eine praxisgerechte Viskosität eingestellt und mit 0,5 Gew.-% N,N-Diallyl-p-dimethylaminobenzolsulfonsäureamid, 0,2 Gew.-% Campherchinon und 0,125 Gew.-% Benzildimethylketal aktiviert. Die aktivierten Mischungen wurden mit einer handelsüblichen Dentallampe (Translux, Fa. Kulzer) zu festen Probekörpern ausgehärtet, an denen die Härteprüfung nach der Wallace-Methode durchgeführt wurde.

Die Wallace-Methode dient zur Bestimmung der Eindruckhärte an Kunststoffen. Ein Vickers-Diamant wird unter einer Vorlast von 1 p auf die Oberfläche aufgebracht und anschließend 60 Sekunden lang mit einer Hauptlast von üblicherweise 100 p beansprucht. Als Maß für den Eindringwiderstand wird die Eindringtiefe des Diamenten in µm unter Hauptlast gemessen. Im Gegensatz zu den Vickers- oder Brinellhärtemessungen, bei denen die Prüfkraft auf die Dimensionen der bleibenden Deformation bezogen wird, erfaßt man mit der Wallace-Methode die elastische und die bleibende Verformung des Kunststoffs. Diese Methode ist zur Charakterisierung von Werkstoffen für Anwendungen auf dem Dentalgebiet besser geeignet als Härteprüfungen, die nur die bleibende Deformation erfassen. Je kleiner die Eindringtiefe Hw ist, um so härter ist das Material.

Tabelle 1 Wallace-Härten

| Monomer | Monomer/TEGDMA* [Gew.-Teile] | Wallace-Härte [µm] |
|---|---|---|
| Monomer aus 1 | 55/45 | 13,0 |
| Monomer aus 2 | 60/40 | 11,9 |
| Monomer aus 3 | 59,9/40,1 | 15,5 |
| Monomer aus 6 | 47,5/52,5 | 12,5 |
| Bis-GMA 1) | 62,0/38,0 | 23,0 |

*TEGDMA = Triethylenglycoldimethacrylat

1) 2,2-Bis[4'-(3'-methacryloyloxy-2'-hydroxy-propoxy)phenyl]propan (Vergleichsbeispiel)

Beispiel 10

Herstellung von Kunststoffzähnen

60 Gew.-Teile einer Monomermischung, die aus 45 Gew.-% Triethylenglykoldimethacrylat und aus 55 Gew.-% des Urethan-Methacrylsäure-Derivates aus Beispiel 1 hergestellt wurde, werden mit 1 Gewichtsteil Dibenzoylperoxid und 40 Gew.-Teilen einer mit 5% 3-Methacryloyloxypropyltrimethoxysilan silanisierten hochdispersen Kieselsäure (BET-Oberfläche : 50 $m^2/g$) vermischt.

Das aktivierte Gemisch wird in eine Zahnform eingespritzt und bei 130°C in 6 Minuten ausgehärtet. Die erhaltenen Kunststoffzähne zeigen eine besonders hohe Abrasionsfestigkeit.

**Patentansprüche**

1. Urethangruppen enthaltende (Meth)-acrylsäurederivate von Tricyclo[5.2.1.$0^{2.6}$]decanen der Formel (I)

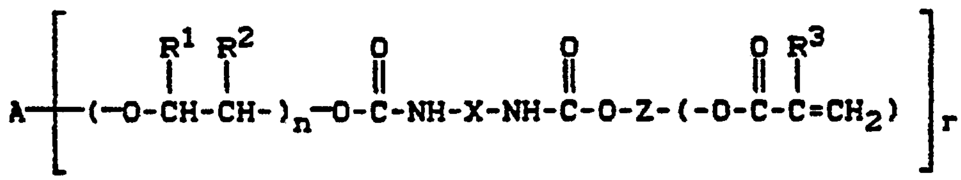 (I),

in der
A ein geradkettiger oder verzweigter, gegebenenfalls 1 bis 3 Sauerstoffbrücken enthaltender, aliphatischer Rest mit 2 bis 20 Kohlenstoff atomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X für die Gruppe

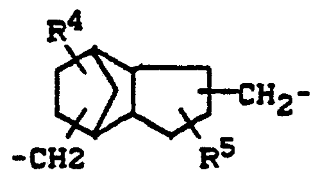

in der
$R^4$ und $R^5$ gleich oder verschieden sind und Wasserstoff, Halogen, Niederalkoxy, Niederalkyl oder Trifluormethyl bedeuten,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
$R^3$ für jede von A ausgehende Kette unabhängig Wasserstoff oder Methyl bedeutet.

2. Urethangruppen enthaltende (Meth)-acrylsäurederivate von Tricyclo[5.2.1.$0^{2.6}$]decanen nach Anspruch 1,
worin
A ein geradkettiger oder verzweigter, gegebenenfalls 1 bis 3 Sauerstoffbrücken enthaltender, aliphatischer Rest mit 3 bis 12 Kohlenstoffatomen, ein aromatischer Rest mit 6 bis 14 Kohlenstoffatomen, ein araliphatischer Rest mit 7 bis 26 Kohlenstoffatomen oder ein cycloaliphatischer Rest mit 6 bis 14 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig einer Zahl von 0 bis 5 bedeutet,
X die Gruppe

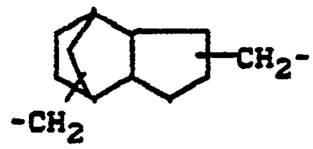

bedeutet,
Z ein zweiwertiger geradkettiger oder verzweigter aliphatischer Kohlenwasserstoff mit 3 bis 10 Kohlenstoffatomen, der gegebenenfalls 1 oder 2 Sauerstoffbrücken enthalten kann und gegebenenfalls durch 1 oder 2 (Methy)-acrylatreste substituiert sein kann, bedeutet, und
$R^3$ für jede von A ausgehende Kette unabhängig Wasserstoff oder Methyl bedeutet.

3. Urethangruppen enthaltende (Meth)acrylsäurederivate von Tricyclo[5.2.1.$0^{2.6}$]decanen nach den Ansprüchen 1 und 2,
worin
A für den 2,2-Bismethylen-butan-1-yl-Rest, Propan-1,2,3-triyl-Rest, 2,2-Bismethylenpropan-1,3-diyl-Rest oder 3(4),8(9)-Bismethylen-tricyclo[5.2.1.$0^{2.6}$]decan-Rest steht,
r für die Anzahl der von A ausgehenden Ketten steht und die Zahl 3 oder 4 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X die Gruppe

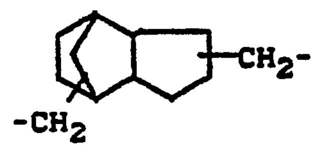

bedeutet,
Z einen zweiwertigen geradkettigen oder verzweigten aliphatischen Kohlenwasserstoffrest mit 3 bis 10 Kohlenstoffatomen, der gegebenenfalls 1 Sauerstoffbrücke enthalten und gegebenenfalls durch 1 (Meth)-acrylatrest substituiert sein kann, bedeutet, und
$R^3$ für jede von A ausgehende Kette unabhängig Wasserstoff oder Methyl bedeutet.

4. Verfahren zur Herstellung von Urethangruppen enthaltenden (Meth)-acrylsäurederivate von Tricyclo[5.2.1.$0^{2.6}$]decanen der Formel (I)

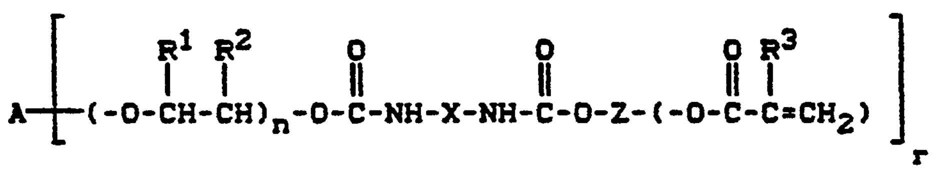 (I),

in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig einer Zahl von 0 bis 5 bedeutet,
X für die Gruppe

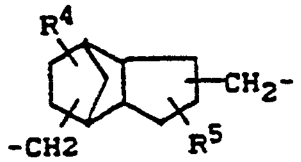 ,

in der
$R^4$ und $R^5$ gleich oder verschieden sind und Wasserstoff, Halogen, Niederalkoxy, Niederalkyl oder Trifluormethyl bedeuten, steht,
Z einen zweitwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 (Meth)-acrylatreste substituiert sein kann, bedeutet, und
$R^3$ für jede von A ausgehende Kette unabhängig Wasserstoff oder Methyl bedeutet,

dadurch gekennzeichnet, daß man einen Hydroxyalkyl-(Meth)-acrylsäureester der Formel (II)

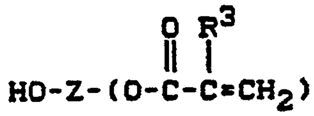

(II),

in der
Z und $R^3$ die obengenannte Bedeutung haben,
mit einem Diisocyanat der Formel (III)

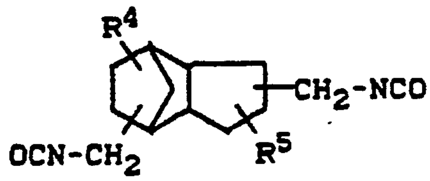

(III),

in der
$R^4$ und $R^5$ die obengenannte Bedeutung haben,
im Molverhältnis von etwa 1:1 bis 1:6 in einem inerten Lösungsmittel in Gegenwart eines Katalysators umsetzt und dann das entstandene Isocyanatourethan nach Entfernen von nicht umgesetzten Diisocyanat mit einem Polyol der Formel (IV)

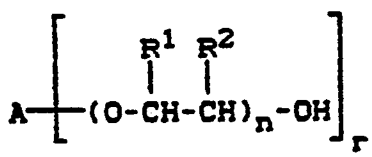

(IV),

in der
A, $R^1$, $R^2$, n und r die obengenannte Bedeutung haben,
im Movlerhältnis von OH-Gruppen zu NCO-Gruppen von etwa 1:1 umsetzt.

5. Verfahren nach Anspruch 4, dadurch gekennzeichnet, daß man die Umsetzung des (Meth)-acrylsäureesters mit dem Diisocyanat des Tricyclo[5.2.1.$0^{2.6}$]decans im Temperaturbereich von 0 bis 120°C durchführt.

6. Verfahren nach den Ansprüchen 4 und 5, dadurch gekennzeichnet, daß man die Umsetzung mit dem Polyol im Temperaturbereich von 0 bis 120°C durchführt.

7. Polymerisat aus Urethangruppen enthaltende (Meth)-acrylsäurederivate von Tricyclo[5.2.1.$0^{2.6}$]-decanen der Formel

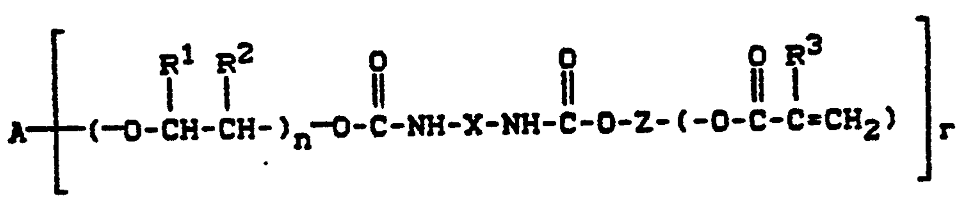

in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X für die Gruppe

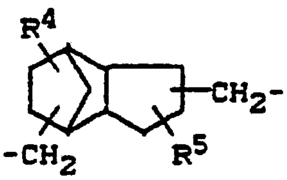

in der
$R^4$ und $R^5$ gleich oder verschieden sind und Wasserstoff, Halogen, Niederalkoxy, Niederalkyl oder Trifluormethyl bedeuten,
steht,
z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
$R^3$ für jede von A ausgehende Kette unabhängig Wasserstoff oder Methyl bedeutet.

8. Verwendung von Urethangruppen enthaltende (Meth)-acrylsäuredeivate von Tricyclo[5.2.1.$0^{2.6}$]-decanen der Formel

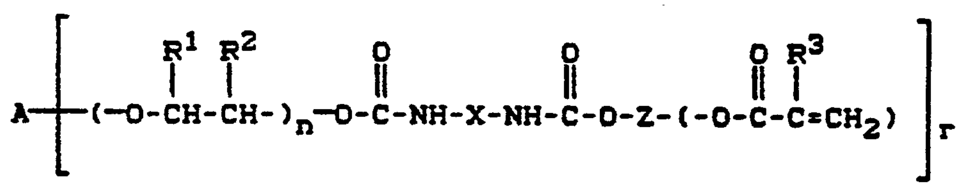

in der
A ein geradkettiger oder verzweigter, gegebenenfalls 1 bis 3 Sauerstoffbrücken enthaltender, aliphatische Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X für die Gruppe

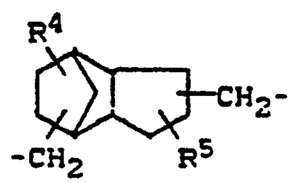

in der
$R^4$ und $R^5$ gleich oder verschieden sind und Wasserstoff, Halogen, Niederalkoxy, Niederalkyl oder Trifluormethyl bedeuten,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
$R^3$ für jede von A ausgehende Kette unabhängig Wasserstoff oder Methyl bedeutet.
in Dentalwerkstoffen.

9. Verwendung nach Anspruch 8, dadurch gekennzeichnet, daß die Urethangruppen enthaltenden (Meth)-acrylsäurederivate in Zahnfüllmassen eingesetzt werden.

10. Verwendung nach Anspruch 8, dadurch gekennzeichnet, daß die Urethangruppen enthaltenden (Meth)-acrylsäurederivate in Beschichtungsmitteln für Zähne eingesetzt werden.

11. Verwendung nach Anspruch 8, dadurch gekennzeichnet, daß die Urethangruppen enthaltenden (Meth)Acrylsäure-Derivate für die Herstellung von Kunststoffzähnen eingesetzt werden.

12. Verwendung von Urethangruppen enthaltenden (Meth)-acrylsäurederivaten der Formel

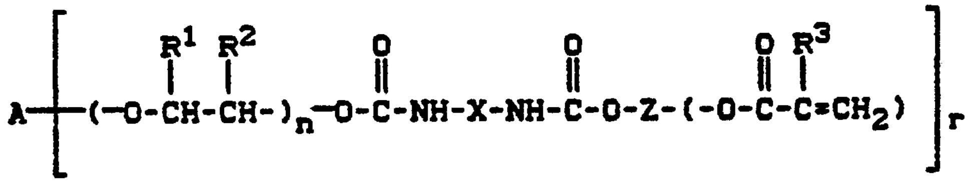

in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X für die Gruppe

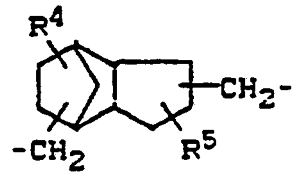

in der
$R^4$ und $R^5$ gleich oder verschieden sind und Wasserstoff, Halogen, Niederalkoxy, Niederalkyl oder Trifluormethyl bedeuten,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
$R^3$ für jede von A ausgehende Kette unabhängig Wasserstoff oder Methyl bedeutet.
zur Herstellung von Dentalwerkstoffen.

13. Dentalwerkstoffe, dadurch gekennzeichnet, daß sie Urethangruppen enthaltende (Meth)-acrylsäurederivate der Formel

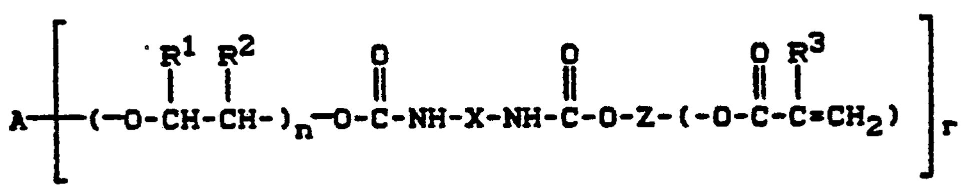

in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und Methyl bedeuten,
n für jede von A ausgehende Kette unabhängig eine Zahl von 0 bis 5 bedeutet,
X für die Gruppe

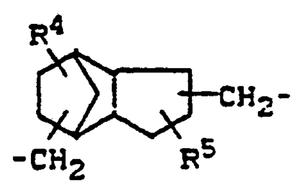

in der
$R^4$ und $R^5$ gleich oder verschieden sind und Wasserstoff, Halogen, Niederalkoxy, Niederalkyl oder Trifluormethyl bedeuten,
steht,

Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
$R^3$ für jede von A ausgehende Kette unabhängig Wasserstoff oder Methyl bedeutet,
enthalten.

14. Dentalwerkstoffe nach Anspruch 13, dadurch gekennzeichnet, daß sie neben Urethangruppen enthaltenden (Meth)-acrylsäurederivaten von Tricyclo[5.2.1.0$^{2.6}$]decanen Comonomere enthalten.

15. Dentalwerkstoffe nach den Ansprüchen 13 und 14, dadurch gekennzeichnet, daß sie neben Urethangruppen enthaltenden (Meth)-acrylsäurederivaten von Tricyclo[5.2.1.0$^{2.6}$]decanen Comonomere, und an sich bekannte Additive und gegebenenfalls Füllstoffe enthalten.

**Claims**

1. (Meth)acrylic acid derivatives, containing urethane groups, of tricyclo[5.2.1.0$^{2.6}$]decanes, of the formula (I)

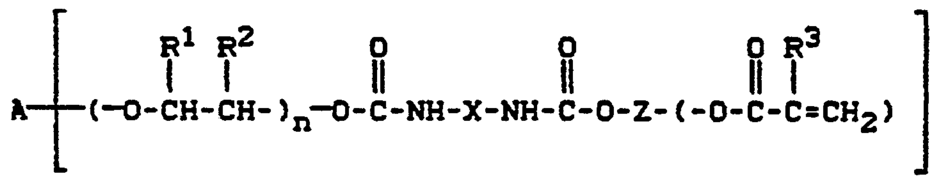

(I)

in which
A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X represents the group

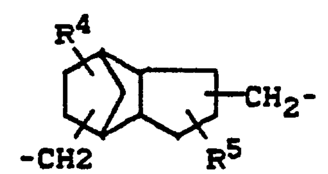

in which
$R^4$ and $R^5$ are identical or different and denote hydrogen, halogen, lower alkoxy, lower alkyl or trifluoromethyl,
Z denotes a divalent straight-chain or branched aliphatic hydrocarbon radical which has 3 to 15 carbon atoms, can optionally contain 1 to 3 oxygen bridges and can optionally be substituted by 1 to 4 additional (meth)acrylate radicals, and
$R^3$ denotes hydrogen or methyl, independently for each chain starting from A.

2. (Meth)acrylic acid derivatives, containing urethane groups, of tricyclo[5.2.1.0$^{2.6}$]decanes, according to Claim 1,
in which
A is a straight-chain or branched aliphatic radical having 3 to 12 carbon atoms and optionally containing 1 to 3 oxygen bridges, an aromatic radical having 6 to 14 carbon atoms, an araliphatic radical having 7 to 26 carbon atoms or a cycloaliphatic radical having 6 to 14 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X denotes the group

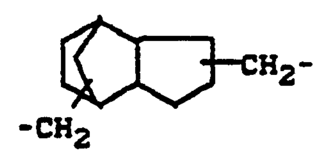

Z denotes a divalent straight-chain or branched aliphatic hydrocarbon which has 3 to 10 carbon atoms,

can optionally contain 1 or 2 oxygen bridges and can optionally be substituted by 1 or 2 (meth)-acrylate radicals, and
$R^3$ denotes hydrogen or methyl, independently for each chain starting from A.

3. (Meth)acrylic acid derivatives, containing urethane groups, of tricyclo[5.2.1.$0^{2.6}$]decanes, according to Claims 1 and 2, in which
A represents the 2,2-bismethylene-butan-1-yl radical, propane-1,2,3-triyl radical, 2,2-bismethylene-propane-1,3-diyl radical or 3(4),8(9)-bismethylene-tri-cyclo[5.2.1.$0^{2.6}$]decane radical,
r represents the number of chains starting from A and denotes the number 3 or 4,
$R^1$ and $R^2$ are identical and denote hydrogen or are different and denote hydrogen and methyl,
n denotes a number from 0 to 5, independently for each chain starting from A,
X denotes the group

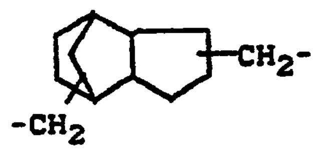

Z denotes a divalent straight-chain or branched aliphatic hydrocarbon radical which has 3 to 10 carbon atoms, can optionally contain 1 oxygen bridge and can optionally be substituted by 1 (meth)acrylate radical, and
$R^3$ denotes hydrogen or methyl, independently for each chain starting from A.

4. Process for the preparation of (meth)acrylic acid derivatives, containing urethane groups, of tricyclo[5.2.1.$0^{2.6}$]-decanes, of the formula (I)

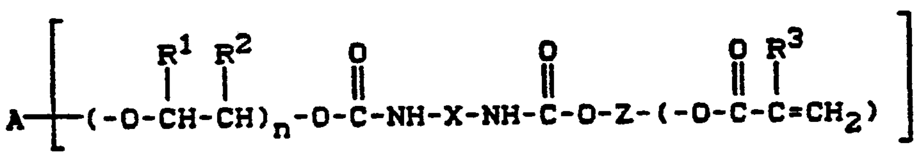 (I),

in which
A is straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X represents the group

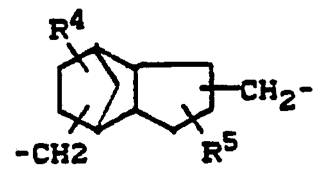

in which
$R^4$ and $R^5$ are identical or different and denote hydrogen, halogen, lower alkoxy, lower alkyl or trifluoromethyl,
Z denotes a divalent straight-chain or branched aliphatic hydrocarbon radical which has 3 to 15 carbon atoms, can optionally contain 1 to 3 oxygen bridges and can optionally be substituted by 1 to 4 (meth)acrylate radicals, and
$R^3$ denotes hydrogen or methyl, independently for each chain starting from A,
characterized in that a hydroxyalkyl (meth)acrylic acid ester of the formula (II)

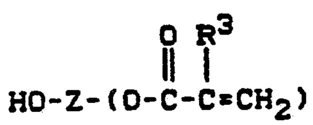 (II),

in which
Z and $R^3$ have the meaning given above, is reacted with a diisocyanate of the formula (III)

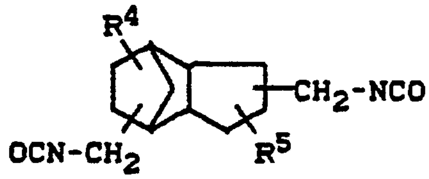

(III),

in which
$R^4$ and $R^5$ have the meaning given above, in a molar ratio of about 1:1 to 1:6 in an inert solvent in the presence of a catalyst and the isocyanatourethane thus formed is then reacted, after removal of unconverted diisocyanate, with a polyol of the formula (IV)

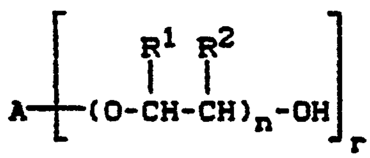

(IV)

in which
A, $R^1$, $R^2$, n and r have the meaning given above, in a molar ratio of OH groups to NCO groups of approximately 1:1.

5. Process according to Claim 4, characterized in that the reaction of the (meth)acrylic acid ester with the diisocyanate of the tricyclo[5.2.1.$0^{2.6}$]decane is carried out in the temperature range from 0 to 120°C.

6. Process according to Claims 4 and 5, characterized in that the reaction with the polyol is carried out in the temperature range from 0 to 120°C.

7. A polymer of (meth)acrylic acid derivatives, containing urethane groups, of tricyclo[5.2.1.$0^{2.6}$]-decanes, of the formula

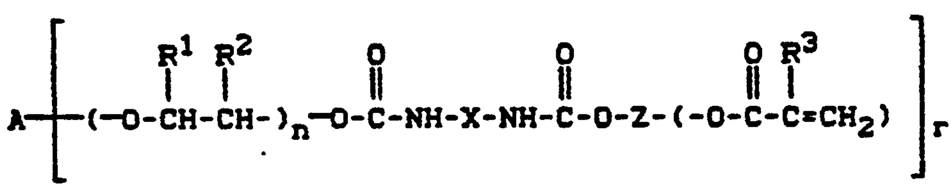

in which
A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X represents the group

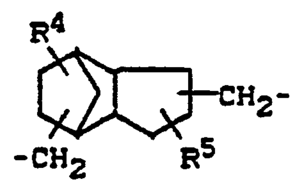

in which
$R^4$ and $R^5$ are identical or different and denote hydrogen, halogen, lower alkoxy, lower alkyl or trifluoromethyl,
Z denotes a divalent straight-chain or branched aliphatic hydrocarbon radical which has 3 to 15 carbon atoms, can optionally contain 1 to 3 oxygen bridges and can optionally be substituted by 1 to 4 additional (meth)acrylate radicals, and
$R^3$ denotes hydrogen or methyl, independently for each chain starting from A.

8. Use of (meth)acrylic acid derivatives, containing urethane groups, of tricyclo[5.2.1.$0^{2.6}$]decanes, of the formula

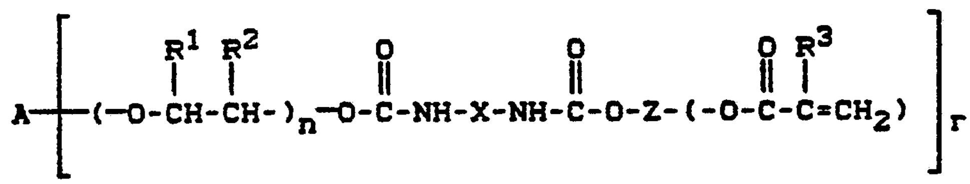

in which
A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X represents the group

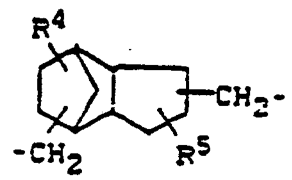

in which
$R^4$ and $R^5$ are identical or different and denote hydrogen, halogen, lower alkoxy, lower alkyl or trifluoromethyl,
Z denotes a divalent straight-chain or branched aliphatic hydrocarbon radical which has 3 to 15 carbon atoms, can optionally contain 1 to 3 oxygen bridges and can optionally be substituted by 1 to 4 additional (meth)acrylate radicals, and
$R^3$ denotes hydrogen or methyl, independently for each chain starting from A, in dental materials.

9. Use according to Claim 8, characterized in that the (meth)acrylic acid derivatives containing urethane groups are used in tooth-filling compositions.

10. Use according to Claim 8, characterized in that the (meth)acrylic acid derivatives containing urethane groups are used in coatings for teeth.

11. Use according to Claim 8, characterized in that the (meth)acrylic acid derivatives containing urethane groups are used for the preparation of plastic teeth.

12. Use of (meth)acrylic acid derivatives, containing urethane groups, of the formula

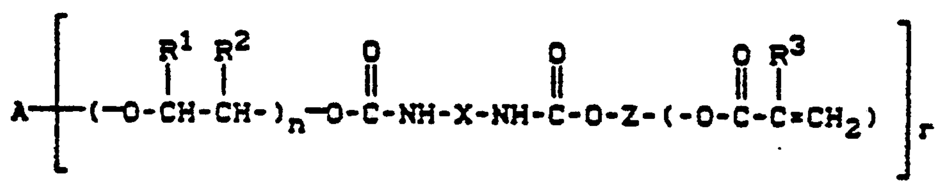

in which
A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X represents the group

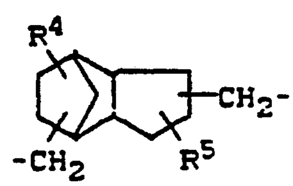

in which
$R^4$ and $R^5$ are identical or different and denote hydrogen, halogen, lower alkoxy, lower alkyl or trifluoromethyl,

Z denotes a divalent straight-chain or branched aliphatic hydrocarbon radical which has 3 to 15 carbon atoms, can optionally contain 1 to 3 oxygen bridges and can optionally be substituted by 1 to 4 additional (meth)acrylate radicals, and
$R^3$ denotes hydrogen or methyl, independently for each chain starting from A, for the preparation of dental materials.

13. Dental materials, characterized in that they contain (meth)acrylic acid derivatives, containing urethane groups, of the formula

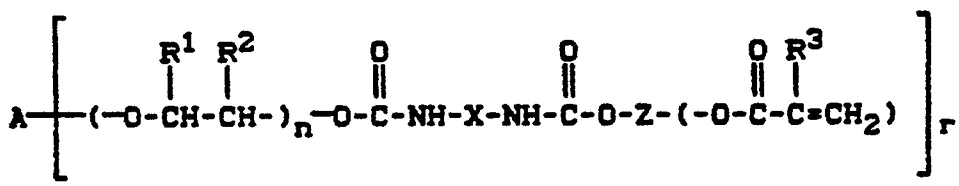

in which
A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,
r represents the number of chains starting from A and denotes a number from 2 to 6,
$R^1$ and $R^2$ are identical and denote hydrogen or are different and denote hydrogen and methyl,
n denotes a number from 0 to 5, independently for each chain starting from A,
X represents the group

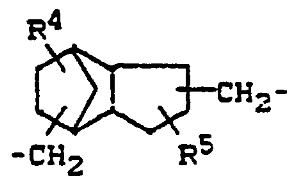

in which
$R^4$ and $R^5$ are identical or different and denote hydrogen, halogen, lower alkoxy, lower alkyl or trifluoromethyl,
Z denotes a divalent straight-chain or branched aliphatic hydrocarbon radical which has 3 to 15 carbon atoms, can optionally contain 1 to 3 oxygen bridges and can optionally be substituted by 1 to 4 additional (meth)acrylate radicals, and
$R^3$ denotes hydrogen or methyl, independently for each chain starting from A.

14. Dental materials according to Claim 13, characterized in that they contain comonomers, in addition to (meth)acrylic acid derivatives, containing urethane groups, of tricyclo[5.2.1.$0^{2.6}$]decanes.

15. Dental materials according to Claims 13 and 14, characterized in that they contain comonomers and additives known per se and, optionally, fillers, in addition to (meth)acrylic acid derivatives, containing urethane groups, of tricyclo[5.2.1.$0^{2.6}$]decanes.

## Revendications

1. Dérivés d'acide (méth)-acrylique porteurs de groupes uréthanne de tricyclo[5.2.1.$0^{2.6}$]décanes de formule (I)

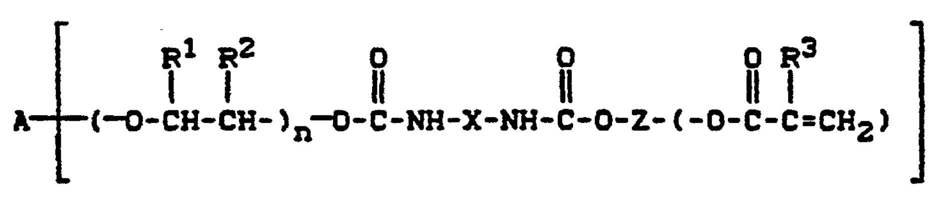 (I),

dans laquelle
A est un reste aliphatique de 2 à 20 atomes de carbone à chaîne droite ou à chaîne ramifiée, contenant éventuellement 1 à 3 ponts oxygène, un reste aromatique de 6 à 24 atomes de carbone, un reste araliphatique de 7 à 26 atomes de carbone ou un reste cycloaliphatique de 6 à 26 atomes de carbone,
r représente le nombre de chaînes partant de A et représente un nombre de 2 à 6,
$R^1$ et $R^2$ sont identiques et représentent l'hydrogène ou sont différents et représentent l'hydrogène et le groupe méthyle,

n pour chaque chaîne partant de A désigne, indépendamment, un nombre de 0 à 5,
X est un groupe de formule

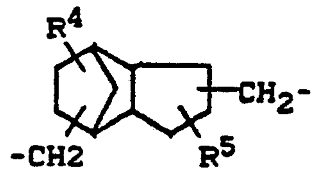

dans laquelle
$R^4$ et $R^5$ sont identiques ou différents et représentent l'hydrogène, un halogène, un groupe alkoxy inférieur, alkyle inférieur ou trifluorométhyle,
Z est un reste d'hydrocarbure aliphatique divalent à chaîne droite ou ramifiée ayant 3 à 15 atomes de carbone, qui peut contenir, le cas échéant, 1 à 3 ponts oxygène et qui peut être éventuellement substitué par 1 à 4 restes (méth)-acrylate supplémentaires, et
$R^3$ représente, indépendamment, pour chaque chaîne partant de A, l'hydrogène ou le groupe méthyle. 2. Dérivés d'acide (méth)-acrylique porteurs de groupes uréthanne de tricyclo[5.2.1.$0^{2.6}$]décanes suivant la revendication 1, dans lesquels
A est un reste aliphatique de 3 à 12 atomes de carbone à chaîne droite ou ramifiée, contenant le cas échéant 1 à 3 ponts oxygène, un reste aromatique de 6 à 14 atomes de carbone, un reste araliphatique de 7 à 26 atomes de carbone ou un reste cycloaliphatique de 6 à 14 atomes de carbone,
r représente le nombre de chaînes partant de A et est un nombre de 2 à 6,
$R^1$ et $R^2$ sont identiques et représentent l'hydrogène ou sont différents et représentent l'hydrogène et le groupe méthyle,
n pour chaque chaîne partant de A représente, indépendamment, un nombre de 0 à 5,
X est le groupe

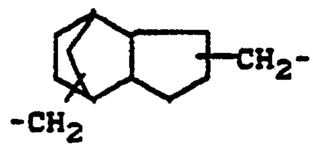

Z est un hydrocarbure aliphatique divalent à chaîne droite ou à chaîne ramifiée ayant 3 à 10 atomes de carbone, qui peut contenir éventuellement 1 ou 2 ponts oxygène et qui peut être substitué, le cas échéant, par 1 ou 2 restes (méth)-acrylate, et
$R^3$ pour chaque chaîne partant de A représente, indépendamment, l'hydrogène ou un groupe méthyle.

3. Dérivés d'acide (méth)-acrylique porteurs de groupes uréthanne de tricyclo[5.2.1.$0^{2.6}$]décanes suivant les revendications 1 et 2, dans lesquels
A représente le reste 2,2-bis-méthylène-butane-1-yle, le reste propane-1,2,3-triyle, le reste 2,2-bis-méthylènepropane-1,3-diyle ou le reste 3(4),8(9)-bis-méthylène-tricyclo[5.2.1.$0^{2.6}$]décane,
r désigne le nombre de chaînes partant de A et représente le nombre 3 ou 4,
$R^1$ et $R^2$ sont identiques et représentent l'hydrogène ou sont différents et représentent l'hydrogène et le groupe méthyle,
n pour chaque chaîne partant de A représente, indépendamment, un nombre de 0 à 5,
X est le groupe

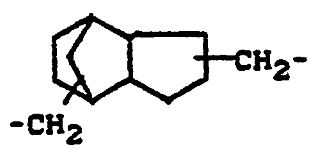

Z est un reste divalent d'hydrocarbure aliphatique à chaîne droite ou à chaîne ramifiée ayant 3 à 10 atomes de carbone, qui peut contenir éventuellement 1 pont oxygène et qui peut être substitué, le cas échéant, par un reste (méth)acrylate, et
$R^3$ pour chaque chaîne partant de A désigne, indépendamment, l'hydrogène ou un groupe méthyle.

4. Procédé de production de dérivés d'acide (méth)-acrylique porteurs de groupes uréthanne de tricyclo[5.2.1.$0^{2.6}$]décanes de formule (I)

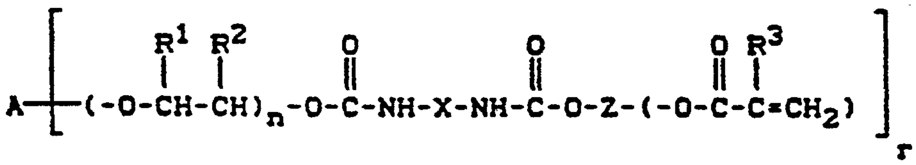 (I),

dans laquelle
A est un reste aliphatique de 2 à 20 atomes de carbone à chaîne droite ou à chaîne ramifiée, contenant éventuellement 1 à 3 ponts oxygène, un reste aromatique de 6 à 24 atomes de carbone, un reste araliphatique de 7 à 26 atomes de carbone ou un reste cycloaliphatique de 6 à 26 atomes de carbone,
r représente le nombre de chaînes partant de A et représente un nombre de 2 à 6,
$R^1$ et $R^2$ sont identiques et représentent l'hydrogène ou sont différents et représentent l'hydrogène et le groupe méthyle,
n pour chaque chaîne partant de A désigne, indépendamment, un nombre de 0 à 5,
X est un groupe de formule

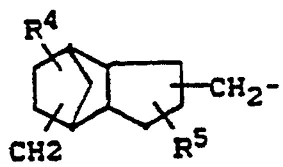 ,

dans laquelle
$R^4$ et $R^5$ sont identiques ou différents et représentent l'hydrogène, un halogène, un groupe alkoxy inférieur, alkyle inférieur ou trifluorométhyle,
Z est un reste d'hydrocarbure aliphatique divalent à chaîne droite ou ramifiée ayant 3 à 15 atomes de carbone, qui peut contenir, le cas échéant, 1 à 3 ponts oxygène et qui peut être éventuellement substitué par 1 à 4 restes (méth)-acrylate, et
$R^3$ représente, indépendamment, pour chaque chaîne partant de A, l'hydrogène ou le groupe méthyle, caractérisé en ce qu'on fait réagir un ester hydroxyalkylique d'acide (méth)-acrylique de formule (II)

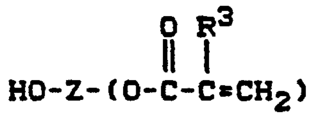 (II),

dans laquelle
Z et $R^3$ ont la définition indiquée ci-dessus, avec un diisocyanate de formule (III)

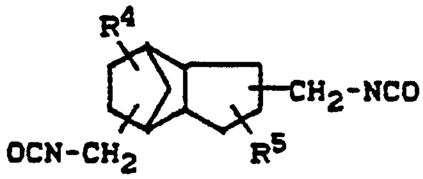 (III),

dans laquelle
$R^4$ et $R^5$ ont la définition indiquée ci-dessus, dans un rapport molaire d'environ 1:1 à 1:6 dans un solvant inerte, en présence d'un catalyseur, puis on fait réagir l'isocyanato-uréthanne produit, après élimination du diisocyanate n'ayant pas réagi, avec un polyol de formule (IV)

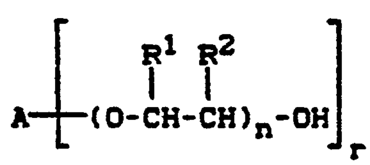 (IV),

dans laquelle
A, $R^1$, $R^2$, n et r ont les définitions indiquées ci-dessus, dans un rapport molaire des groupes OH aux groupes NCO d'environ 1:1.

5. Procédé suivant la revendication 4, caractérisé en ce qu'on effectue la réaction de l'ester d'acide

(méth)-acrylique avec le diisocyanate de tricyclo[5.2.1.0$^{2.6}$]décane dans la plage de températures de 0 à 120°C.

6. Procédé suivant les revendications 4 et 5, caractérisé en ce qu'on conduit la réaction avec le polyol dans la plage de températures de 0 à 120°C.

7. Produit de polymérisation obtenu à partir de dérivés d'acide (méthy)-acrylique porteurs de groupes uréthanne de tricyclo[5.2.1.0$^{2.6}$]décanes de formule

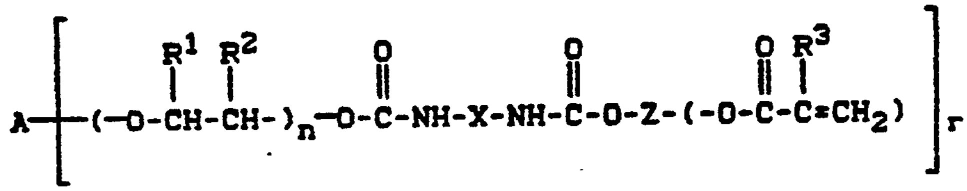

dans laquelle
A est un reste aliphatique de 2 à 20 atomes de carbone à chaîne droite ou à chaîne ramifiée, contenant éventuellement 1 à 3 ponts oxygène, un reste aromatique de 6 à 24 atomes de carbone, un reste araliphatique de 7 à 26 atomes de carbone ou un reste cycloaliphatique de 6 à 26 atomes de carbone,
r représente le nombre de chaînes partant de A et représente un nombre de 2 à 6,
$R^1$ et $R^2$ sont identiques et représentent l'hydrogène ou sont différents et représentent l'hydrogène et le groupe méthyle,
n pour chaque chaîne partant de A désigne, indépendamment, un nombre de 0 à 5,
X est un groupe de formule

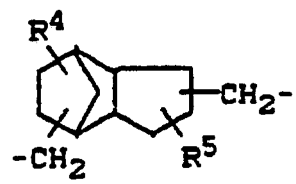

dans laquelle
$R^4$ et $R^5$ sont identiques ou différents et représentent l'hydrogène, un halogène, un groupe alkoxy inférieur, alkyle inférieur ou trifluorométhyle,
Z est un reste d'hydrocarbure aliphatique divalent à chaîne droite ou ramifiée ayant 3 à 15 atomes de carbone, qui peut contenir, le cas échéant, 1 à 3 ponts oxygène et qui peut être éventuellement substitué par 1 à 4 restes (méth)-acrylate supplémentaires, et
$R^3$ représente, indépendamment, pour chaque chaîne partant de A, l'hydrogène ou le groupe méthyle.

8. Utilisation de dérivés d'acide (méth)acrylique porteurs de groupes uréthanne de tricyclo[5.2.1.0$^{2.6}$]-décanes de formule

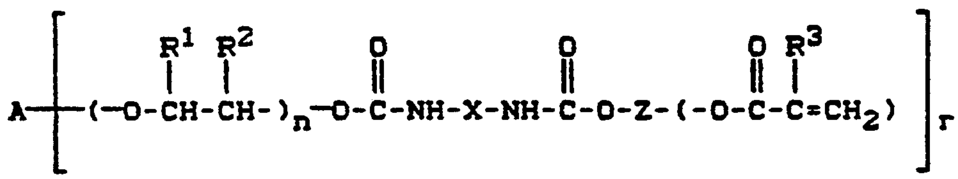

dans laquelle
A est un reste aliphatique de 2 à 20 atomes de carbone à chaîne droite ou à chaîne ramifiée, contenant éventuellement 1 à 3 ponts oxygène, un reste aromatique de 6 à 24 atomes de carbone, un reste araliphatique de 7 à 26 atomes de carbone ou un reste cycloaliphatique de 6 à 26 atomes de carbone,
r représente le nombre de chaînes partant de A et représente un nombre de 2 à 6,
$R^1$ et $R^2$ sont identiques et représentent l'hydrogène ou sont différents et représentent l'hydrogène et le groupe méthyle,
n pour chaque chaîne partant de A désigne, indépendamment, un nombre de 0 à 5,
X est un groupe de formule

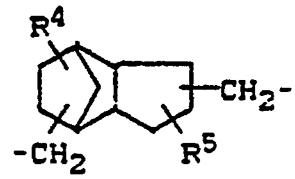

dans laquelle
$R^4$ et $R^5$ sont identiques ou différents et représentent l'hydrogène, un halogène, un groupe alkoxy inférieur, alkyle inférieur ou trifluorométhyle,
Z est un reste d'hydrocarbure aliphatique divalent à chaîne droite ou ramifiée ayant 3 à 15 atomes de carbone, qui peut contenir, le cas échéant, 1 à 3 ponts oxygène et qui peut être éventuellement substitué par 1 à 4 restes (méth)-acrylate supplémentaires, et
$R^3$ représente indépendamment, pour chaque chaîne partant de A, l'hydrogène ou le groupe méthyle dans des matériaux à usage dentaire.

9. Utilisation suivant la revendication 8, caractérisée en ce que les dérivés d'acide (méth)-acrylique porteurs de groupes uréthanne sont incorporés dans des mélanges pour obturation dentaire.

10. Utilisation suivant la revendication 8, caractérisée en ce que les dérivés d'acide (méth)-acrylique porteurs de groupes uréthanne sont incorporés dans des compositions pour le revêtement de dents.

11. Utilisation suivant la revendication 8, caractérisée en ce que les dérivés d'acide (méth)-acrylique porteurs de groupes uréthanne sont utilisés pour la production de dents artificielles.

12. Utilisation de dérivés d'acide (méth)-acrylique porteurs de groupes uréthanne de formule

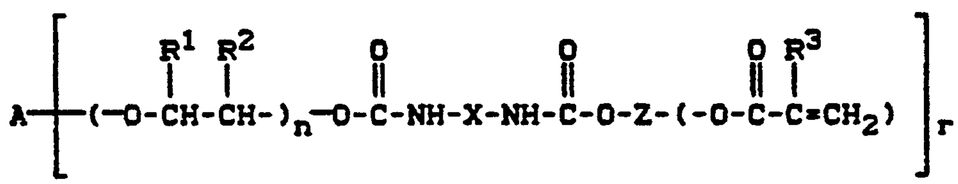

dans laquelle
A est un reste aliphatique de 2 à 20 atomes de carbone à chaîne droite ou à chaîne ramifiée, contenant éventuellement 1 à 3 ponts oxygène, un reste aromatique de 6 à 24 atomes de carbone, un reste araliphatique de 7 à 26 atomes de carbone ou un reste cycloaliphatique de 6 à 26 atomes de carbone,
r représente le nombre de chaînes partant de A et représente un nombre de 2 à 6,
$R^1$ et $R^2$ sont identiques et représentent l'hydrogène ou sont différents et représentent l'hydrogène et le groupe méthyle,
n pour chaque chaîne partant de A désigne, indépendamment, un nombre de 0 à 5,
X est un groupe de formule

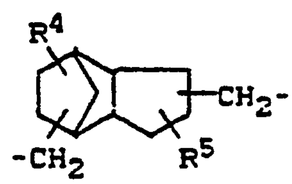

dans laquelle
$R^4$ et $R^5$ sont identiques ou différents et représentent l'hydrogène, un halogène, un groupe alkoxy inférieur, alkyle inférieur ou trifluorométhyle,
Z est un reste d'hydrocarbure aliphatique divalent à chaîne droite ou ramifiée ayant 3 à 15 atomes de carbone, qui peut contenir, le cas échéant, 1 à 3 ponts oxygène et qui peut être éventuellement substitué par 1 à 4 restes (méth)-acrylate supplémentaires, et
$R^3$ représente, indépendamment, pour chaque chaîne partant de A, l'hydrogène ou le groupe méthyle pour la production de matériaux à usage dentaire.

13. Matériaux à usage dentaire, caractérisés en ce qu'ils contiennent des dérivés d'acide (méth)-acrylique porteurs de groupes uréthanne, de formule

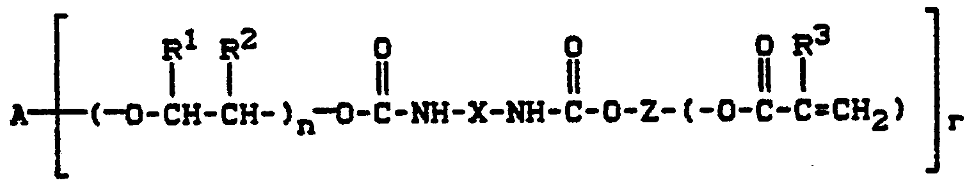

dans laquelle
A est un reste aliphatique de 2 à 20 atomes de carbone à chaîne droite ou à chaîne ramifiée, contenant éventuellement 1 à 3 ponts oxygène, un reste aromatique de 6 à 24 atomes de carbone, un reste araliphatique de 7 à 26 atomes de carbone ou un reste cycloaliphatique de 6 à 26 atomes de carbone,
r représente le nombre de chaînes partant de A et représente un nombre de 2 à 6,
$R^1$ et $R^2$ sont identiques et représentent l'hydrogène ou sont différents et représentent l'hydrogène et le groupe méthyle,
n pour chaque chaîne partant de A désigne, indépendamment, un nombre de 0 à 5,
X est un groupe de formule

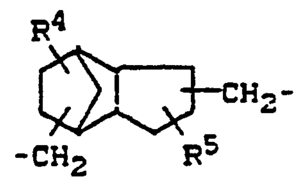

dans laquelle
$R^4$ et $R^5$ sont identiques ou différents et représentent l'hydrogène, un halogène, un groupe alkoxy inférieur, alkyle inférieur ou trifluorométhyle,
Z est un reste d'hydrocarbure aliphatique divalent à chaîne droite ou ramifiée ayant 3 à 15 atomes de carbone, qui peut contenir, le cas échéant, 1 à 3 ponts oxygène et qui peut être éventuellement substitué par 1 à 4 restes (méth)-acrylate supplémentaires, et
$R^3$ représente indépendamment, pour chaque chaîne partant de A, l'hydrogène ou le groupe méthyle.

14. Matériaux à usage dentaire suivant la revendication 13, caractérisés en ce qu'ils contiennent des comonomères en plus de dérivés d'acide (méth)-acrylique porteurs de groupes uréthanne de tricyclo-[5.2.1.0$^{2.6}$]décanes.

15. Matériaux à usage dentaire suivant les revendications 13 et 14, caractérisés en ce qu'ils contiennent, à côté de dérivés d'acide (méth)-acrylique porteurs de groupes uréthanne de tricyclo[5.2.1.0$^{2.6}$]-décanes, des comonomères et des additifs, et le cas échéant des charges, de type connu.